# FASCICULE DE BREVET EUROPEEN

(11) **EP 0 516 540 B1**
(45) Date de publication et mention de la délivrance du brevet: **29.01.1997**
(21) Numéro de dépôt: 92401456.6
(22) Date de dépôt: 27.05.1992
(51) Int. Cl.: C12Q 1/70, C12Q 1/68, C07H 21/00, C12P 19/34, C12N 15/48

(54) **Oligonucléotides utilisables comme réactifs pour la détection in vitro des infections à rétrovirus de type HIV et procédé de détection de rétrovirus de type HIV**
Oligonukleotide für den in vitro Nachweis von HIV-Retroviren-Infektionen und Verfahren für den Nachweis von HIV-Retroviren
Oligonucleotides useful as reactants for the in vitro detection of HIV retrovirus infections and HIV retrovirus detection method

(30) Priorité: 31.05.1991 FR 9106579
(43) Date de publication de la demande: 02.12.1992
(73) Titulaire: CIS BIO INTERNATIONAL, F-91400 Saclay (FR)
(72) Inventeur: Sauvaigo, Sylvie, F-38000 Grenoble (FR); Fouque, Brigitte, F-38170 Seyssinet (FR)
(74) Mandataire: Orès, Bernard

(56) Documents cités:
- EP-A- 0 229 701
- EP-A- 0 269 445
- EP-A- 0 272 098
- EP-A- 0 370 694
- EP-A- 0 386 563
- EP-A- 0 403 333
- EP-A- 0 404 625
- EP-A- 0 439 222
- EP-A- 0 469 610
- PROCEEDINGS OF THE NATIONAL ACADEMY OF SCIENCES OF USA. vol. 86, Avril 1989, WASHINGTON US pages 2423 - 2427; D. J. KEMP ET AL.: 'Colorimetric detection of specific DNA segments amplified by polymerase chain reactions'
- SCIENCE. vol. 239, 15 Janvier 1988, LANCASTER, PA US pages 295 - 297; CHIN-YIH OU ET AL.
- Journal of Clinical Microbiology, 31(5), 1993, 1066-74
- AIDS, 6, 1992, 963-8

## Description

La présente invention est relative à des oligonucléotides, utilisables comme réactifs pour la détection *in vitro* des infections à rétrovirus de type HIV (HIV-1 et/ou HIV-2).

L'isolement et la caractérisation du rétrovirus de type HIV-1 a été décrit dans la Demande de Brevet européen n° 138 667 et le rétrovirus de type HIV-2 a été décrit dans la Demande de Brevet européen n° 239 425.

Ces deux rétrovirus ont pour cibles préférentielles les lymphocytes T4 humains et présentent un effet cytopathogène à l'égard de ces lymphocytes, qui entraîne une lymphadénopathie ou un syndrome d'immunodéficience acquise (SIDA) et des nombreuses infections opportunistes associées.

Ces deux virus présentent une homologie de 60 % sur l'ensemble de l'acide nucléique des gènes *gag* et *pol* et de 30-40 % pour les autres gènes viraux et le LTR.

Le diagnostic et/ou la détection fiable des rétrovirus de type HIV est important, notamment pour suivre les individus à haut risque et aussi pour essayer de prévenir la transmission de la maladie.

Les tests de diagnostic sérologiques ou immunologiques de l'infection à virus de type HIV se fondent principalement sur la détection des anticorps par des méthodes immunoenzymatiques s'appuyant sur la technique ELISA. Ces techniques mettent en évidence des marqueurs indirects de l'infection au virus HIV et ne fournissent donc pas de résultats interprétables (faux négatifs notamment), dans les cas où une détection précoce est nécessaire, en particulier dans le cas des enfants nés de mères séropositives ou dans le cas des individus à haut risque, avant une séroconversion, par exemple.

Des détections directes, qui peuvent être interprétables précocement, ont été préconisées ; on peut citer notamment le dépistage d'antigènes circulants ou encore l'isolement du virus à partir des lymphocytes d'un patient ; toutefois cette approche n'est pas adaptée à une utilisation en routine, notamment dans des laboratoires d'analyse médicale.

C'est pourquoi d'autres tests ont été développés, et associent notamment la détection du génome HIV par hybridation moléculaire, à une technique d'amplification génomique.

La Demande de Brevet français 2 647 810, aux noms de l'INSTITUT PASTEUR et de l'INSERM, décrit des amorces oligonucléotidiques comprenant une vingtaine de bases pour l'amplification du génome des rétrovirus du type HIV-2 et SIV et leurs applications au diagnostic *in vitro* des infections dues à ces virus. Cette Demande décrit plus particulièrement une série de couples d'amorces qui sont choisies de manière à ce que les fragments d'ADN synthétisés couvrent les régions P₁ à P₂, LTR₁ à *pol*₂, P₂ à P₇, P₇ à P₈ et P₈ à LTR₂ desdits virus.

La Demande de Brevet français 2 652 091, aux noms de l'INSTITUT PASTEUR et de l'INSERM, décrit des séquences nucléotidiques issues du génome des rétrovirus du type HIV-1, HIV-2 et SIV et leurs applications notamment en tant qu'amorces pour l'amplification des génomes de ces rétrovirus et pour le diagnostic *in vitro* des infections dues à ces virus. Les oligonucléotides de cette Demande sont notamment comprises dans une séquence commune aux génomes des virus HIV-1, HIV-2 et SIV située dans les gènes *gag*, *vpr* et *pol*.

Cependant comme le précise cette Demande, ainsi que J.J. SNINSKY et S. KWOK [Detection of human immunodeficiency viruses by the polymerase chain reaction, (Arch. Path. Lab. Med., 1990, 114, 259-262)], le choix des amorces est crucial pour obtenir une bonne sensibilité et un bon rendement de l'amplification ; en effet, la sensibilité de la PCR (amplification par réaction de polymérisation en chaîne) dépend de la spécificité avec laquelle les amorces initient la polymérisation de l'acide nucléique, à partir de la séquence cible, par rapport à des régions non spécifiques de l'acide nucléique.

La Demande de Brevet EP 370 694, au nom de CETUS CORP., décrit un procédé de détection d'une séquence d'acide nucléique double-brin, comprenant, pour la détection, un moyen de capture de la séquence en phase solide.

Cette méthode comprend une amplification en présence de deux amorces oligonucléotidiques, au moins l'une desdites amorces étant marquée avec un ligand de liaison spécifique ; les produits d'extension sont séparés et détectés par une sonde appropriée. Une étape au cours de laquelle le ligand de liaison spécifique de la première amorce est complexé avec un récepteur fixé sur un support solide est nécessaire (étape de capture). Cette méthode présente plusieurs variantes, selon le moment où est effectuée la capture : soit la capture est réalisée avant l'amplification, soit la capture est réalisée après l'amplification.

L'exemple 1 de cette Demande est une application de la méthode à la détection du virus HIV-1 :
. les amorces présentent les séquences suivantes :
   X-TTTGGTCCTTGTCTTATGTCCAGAATGC et X-ATAATCCACCTATCCCAGTAGGAGAAAT, dans lesquelles X représente un bras biotine tétraéthylèneglycol ;
et permettent l'amplification d'un fragment de 180 nucléotides de la région *gag* du virus HIV-1.

D'autres facteurs peuvent également intervenir dans la sensibilité de la méthode d'amplification :
- les variations de concentration en enzyme, amorces et cations métalliques (MgCl₂) peuvent notamment avoir un effet important sur le rendement en produit de la PCR ;
- la longueur des amorces, les températures et les temps d'hybridation, ainsi que les étapes d'extension de l'acide nucléique de chaque cycle peuvent également affecter la sensibilité et la spécificité des amplifications.

Il ressort de ces différents documents qu'une amplification sensible et présentant un bon rendement doit combiner des réactifs très spécifiques (amorces) et un protocole d'amplification particulièrement bien adapté.

Les différentes méthodes énumérées ci-dessus présentent un certain nombre d'inconvénients :
- le test immunologique manque de sensibilité, ceci étant sans doute dû à l'infection latente et/ou à la séquestration de l'antigène viral dans des immuns complexes circulants ;
- l'hybridation *in situ* et l'analyse par Southern blot ont été utilisées, avec un succès sporadique en raison du faible nombre de cellules mononucléaires infectées du sang périphérique ;
- l'utilisation de l'amplification a amélioré la sensibilité du test de détection des virus HIV ; toutefois les amorces décrites aussi bien dans les Demandes Pasteur et al. précitées que dans l'article paru dans J. Infect. Dis., en 1988 (158, 6, 1170-1176), au nom de M. RAYFIELD et al., (amorces et sondes, sélectionnées au niveau du gène *gag* (p24)) ont l'inconvénient :
   . de présenter des réactions croisées (séquences communes),
   . de ne pas fournir de rendements suffisants pour la détection de très petites quantités de virus,
   . de ne pas permettre une discrimination poussée entre les infections à HIV-1 et à HIV-2, ou
   . de ne pas détecter tous les variants de HIV connus ou inconnus dans la mesure où dans les conditions drastiques d'hybridation préconisées, ces amorces, d'une longueur d'une vingtaine de bases, ont été sélectionnées de façon à obtenir 100 % d'homologie avec la cible et ne permettent pas ou peu l'hybridation avec les acides nucléiques de HIV ayant subi des modifications de séquence (mutations par exemple).

La présente invention s'est en conséquence donné pour but de fournir des réactifs de diagnostic (amorces et sondes), qui répondent mieux aux nécessités de la pratique que les réactifs proposés dans l'Art antérieur, notamment en ce que lesdits réactifs permettent l'obtention de rendements importants en séquences cibles, s'hybrident notamment avec des régions hautement conservées des HIV, tout en étant insensibles aux variations du génome de ces virus et en permettant une discrimination fine des HIV-1 et des HIV-2.

La présente invention a pour objet des oligonucléotides, comprenant environ 25 à 35 nucléotides, pour le diagnostic différentiel des virus HIV-1 et HIV-2, caractérisés en ce qu'ils sont sélectionnés parmi au moins l'une des séquences suivantes :
a) les séquences homologues issues du gène *tat* des HIV-1 et plus particulièrement des virus HIV-1 BRU, HIV-1 ARV, HIV-1 HXB2, HIV-1 BH5, HIV-1 ELI, HIV-1 NDK, HIV-1 MAL, HIV-1 SF2 et les séquences complémentaires de ces séquences, qui correspondent aux positions 5419-5446, 5447-5474, 5533-5560, 5563-5590 du génome du virus HIV-1 BRU ;
b) les séquences homologues issues du gène *tat* des HIV-2 et les séquences complémentaires de ces séquences, qui correspondent aux positions 8311-8338, 8363-8390, 8426-8453, 8480-8507 du génome du virus HIV-2 ROD ;
c) les séquences homologues issues du gène *pol* des HIV-1 et plus particulièrement des virus HIV-1 BRU, HIV-1 ARV, HIV-1 HXB2, HIV-1 BH5, HIV-1 ELI, HIV-1 NDK, HIV-1 MAL, HIV-1 SF2 et les séquences complémentaires de ces séquences, qui correspondent aux positions 3208-3235, 3436-3463, 3647-3675, 3739-3766 du génome du virus HIV-1 BRU ;
d) les séquences homologues issues du gène *pol* des HIV-2 et les séquences complémentaires de ces séquences, qui correspondent aux positions 3141-3168, 3241-3268, 3651-3678, 3693-3720 du génome du virus HIV-2 ROD ; et
e) les séquences homologues issues du gène *gag* dans HIV-1 et plus particulièrement des virus HIV-1 BRU, HIV-1 ARV, HIV-1 HXB2, HIV-1 BH5, HIV-1 ELI, HIV-1 NDK, HIV-1 MAL, HIV-1 SF2 et les séquences complémentaires de ces séquences et qui correspondent aux positions 882-909, 949-976 et 1047-1074 du génome du virus HIV-1 BRU.

Au sens de la présente invention, "séquence homologue" englobe non seulement les séquences identiques ou complémentaires aux fragments de gène sus-mentionnés, mais également celles n'en différant que par la substitution, la délétion ou l'addition d'un petit nombre de nucléotides, à condition que les séquences ainsi modifiées aient une spécificité d'hybridation équivalente à celle des oligonucléotides décrits ci-dessus.

De même, on entend par "séquence complémentaire", non seulement les séquences strictement complémentaires des fragments de gène conformes à l'invention, mais également des séquences modifiées comme indiqué précédemment, possédant une spécificité d'hybridation équivalente à celle desdites séquences strictement complémentaires.

On définit notamment, dans la présente invention, les conditions d'hybridation comme suit :

KCl 50mM, Tris HCl pH 8,3 10mM, MgCl₂, 2,5mM, gélatine, 0,1 mg/ml, en présence de 20 à 50 pmoles de chacun des oligonucléotide conforme à l'invention.

Les oligonucléotides conformes à l'invention, d'une longueur supérieure à 25 bases, ont l'avantage :
- de s'hybrider et de permettre la PCR avec la séquence HIV convenable même si celle-ci a subi plusieurs mutations,
- de permettre d'effectuer les amplifications dans des conditions peu drastiques, ce qui permet aux oligonucléotides conformes à l'invention de détecter tous les variants tout en restant spécifiques de l'espèce recherchée,
- d'augmenter ainsi l'éventail des souches d'HIV détectables, tout en restant spécifiques.

Conformément à l'invention, lesdits oligonucléotides répondent à l'une quelconque des formules suivantes :

(1) 5' CAG TAG ATC CTA GAC TAG AGC CCT GGA A (SEQUENCE ID N° 1)

(2) 5' GCA TCC AGG AAG TCA GCC TAG GAC TGC T (SEQUENCE ID N° 2)

(3) 5' TTC CTG CCA TAG GAG ATG CCT AAG GCT T (SEQUENCE ID N° 3)

(4) 5' TGA GGA GGT CTT CGT CGC TGT CTC CGC T (SEQUENCE ID N° 4)

(5) 5' TGC CCA CAC TAA TGA TGT AAA ACA ATT A (SEQUENCE ID N° 5)

(6) 5' AGG AGC AGA AAC TTT CTA TGT AGA TGG G (SEQUENCE ID N° 6)

(7) 5' ATT CAC TTT TAT CTG GTT GTG CTT GAA T (SEQUENCE ID N° 7)

(8) 5' ATT TCC TCC AAT TCC TTT GTG TGC TGG T (SEQUENCE ID N° 8)

(9) 5' ATA TCC ACA AGG ACC GGG GAC AGC CAG C (SEQUENCE ID N° 9)

(10) 5' TGG AAG CAA CGG TGG AGA CAG ATA CTG G (SEQUENCE ID N° 10)

(11) 5' TAG TCT GGT CAA GAG GCG AAT CAG CTG G (SEQUENCE ID N° 11)

(12) 5' GAT GAG TTG GAG GGT CAG GAA GCT CCT G (SEQUENCE ID N° 12)

(13) 5' TCA ATG ACA TCC AGA AGC TAG TGG GTG T (SEQUENCE ID N° 13)

(14) 5' GAC ACT CAC AGA AGA AGT ACA GTG GAC A (SEQUENCE ID N° 14)

(15) 5' ATA GGA TCC CCT ACC AGG TTA AAC GCT A (SEQUENCE ID N° 15)

(16) 5' CTA TTG CAG GAT CCA TCT GTG TAG AAG G (SEQUENCE ID N° 16)

(17) 5' GAT TTA AAC ACC ATG CTA AAC ACA GTG G (SEQUENCE ID N° 17)

(18) 5' TCA ATG AGG AAG CTG CAG AAT GGG ATA G (SEQUENCE ID N° 18)

(19) 5' TTT GTT CCT GAA GGG TAC TAG TAG TTC C (SEQUENCE ID N° 19)

Les oligonucléotides (1) à (8) s'hybrident spécifiquement avec l'HIV-1 et peuvent notamment être modifiés, de manière à présenter le minimum d'homologie avec les séquences correspondantes du virus HIV-2 ROD.

Les oligonucléotides (9) à (16) s'hybrident spécifiquement avec l'HIV-2 et peuvent notamment être modifiés, de manière à présenter le minimum d'homologie avec les séquences correspondantes de l'HIV-1 BRU.

Les oligonucléotides (17) à (19) s'hybrident spécifiquement avec l'HIV-1.

Les positions de ces séquences dans le génome du virus HIV-1 BRU ou dans le génome du virus HIV-2 ROD sont rappelées dans les Tableaux ci-après.
* virus HIV-1 :

| tat de HIV-1 BRU (n)* | pol de HIV-1 BRU (n)* | gag de HIV-1 BRU (n)* |
|---|---|---|
| positions 5419-5446 (1) | positions 3208-3235 (5) | positions 882-906 (17) |
| positions 5447-5474 (2) | positions 3436-3463 (6) | positions 949-976 (18) |
| positions 5533-5560 (3) | positions 3647-3675 (7) | positions 1047-1074 (19) |
| positions 5563-5590 (4) | positions 3739-3766 (8) | |

| | | |
|---|---|---|
| * correspond au numéro du nucléotide | | |

* virus HIV-2 :

| tat de HIV-2 ROD (n)* | pol de HIV-2 ROD (n)* |
|---|---|
| positions 8311-8338 (9) | positions 3141-3168 (13) |
| positions 8363- 8390 (10) | positions 3241-3268 (14) |
| positions 8426-8453 (11) | positions 3651-3678 (15) |
| positions 8480-8507 (12) | positions 3693-3720 (16) |

| | |
|---|---|
| * correspond au numéro du nucléotide | |

La présente invention a également pour objet un réactif de diagnostic pour la détection d'au moins un rétrovirus de type HIV (HIV-1 et/ou HIV-2), caractérisé en ce qu'il comprend au moins un oligonucléotide conforme à l'invention, éventuellement marqué de manière appropriée.

Selon un mode de réalisation avantageux dudit réactif, il correspond à des paires d'amorces pour la synthèse d'un fragment nucléique d'HIV.

Conformément à ce mode de réalisation, ledit réactif est notamment constitué par l'une des paires d'amorces suivantes :
. paire A : oligonucléotide (1) et oligonucléotide (4),
. paire B : oligonucléotide (5) et oligonucléotide (8),
. paire C : oligonucléotide (9) et oligonucléotide (12),
. paire D : oligonucléotide (13) et oligonucléotide (16),
. paire E : oligonucléotide (2) et oligonucléotide (3),
. paire F : oligonucléotide (6) et oligonucléotide (7),
. paire G : oligonucléotide (10) et oligonucléotide (11),
. paire H : oligonucléotide (14) et oligonucléotide (15),
. paire I : oligonucléotide (17) et oligonucléotide (20), de formule 5' TTT GGT CCC TGT CTT ATG TCC AAA ATG C (SEQUENCE ID N°20)
. paire J : oligonucléotide (18) et oligonucléotide (19).

Selon un autre mode de réalisation avantageux dudit réactif, il correspond à une sonde de détection ou de révélation d'un fragment nucléique d'HIV et est marqué de manière convenable.

On peut citer, à titre d'exemple, comme marqueurs convenables, les isotopes radioactifs, les enzymes appropriées, les fluorochromes ou les marqueurs chimiques appropriés et les haptènes, les anticorps, ou les analogues de base.

En effet, de tels oligonucléotides peuvent être utilisés soit comme amorce, soit comme sonde, dans une méthode d'hybridation ou dans une méthode d'amplification convenable [technique PCR (Demande européenne CETUS n°201 184) ; technique dite de la Qβ replicase (Biotechnol., 1988, 6, 1197) ; technique à la T7 ARN polymérase (Demande internationale PCT WO89/01050)] et également dans celle décrite dans la Demande Internationale PCT/FR90/00410.

Les oligonucléotides conformes à l'invention ont l'avantage :
- d'être spécifiques des virus du type HIV-1 ou HIV-2,
- d'être insensibles aux variations génomiques de ces virus, et
- de permettre une discrimination poussée et simultanée (marqueurs distincts, longueur des séquences cibles obtenues, par exemple) entre les infections dues à des virus HIV-1 et celles dues à des virus HIV-2.

La présente invention a également pour objet un procédé de détection d'un virus HIV par la détection de sa séquence d'acide nucléique dans un échantillon biologique, caractérisé en ce qu'il comprend, après une mise en solution appropriée de l'échantillon biologique pour extraire l'acide nucléique :
A. une étape dans laquelle on met en contact l'échantillon biologique avec au moins un réactif de diagnostic choisi dans le groupe qui comprend les réactifs conformes à l'invention et tout autre réactif apte à s'hybrider avec la séquence à détecter, et
B. une étape dans laquelle on détecte par tout moyen approprié le/les produits résultant de l'interaction séquence nucléotidique d'HIV éventuellement présente-réactif de diagnostic.

Selon un mode de mise en oeuvre avantageux dudit procédé le réactif de diagnostic de l'étape A est choisi dans le groupe qui comprend les paires d'amorces dont au moins l'une d'entre elles est un oligonucléotide conforme à l'invention et les paires d'amorces convenables pour l'amplification de la séquence nucléotidique d'HIV c'est-à-dire apte à s'hybrider avec la séquence à amplifier, lequel procédé permet l'obtention du produit d'amplification de la séquence nucléotidique à détecter.

Selon une disposition avantageuse de ce mode de mise en oeuvre, les produits d'amplification obtenus en A sont détectés par hybridation entre lesdits produits d'amplification et un réactif de diagnostic conforme à l'invention, marqué de manière approprié.

Selon un autre mode de mise en oeuvre dudit procédé, le réactif de diagnostic de l'étape A est une sonde de détection ou de révélation conforme à l'invention et permet l'obtention d'hybrides entre la séquence nucléotidique à détecter et ladite sonde.

En variante, lorsque le procédé conforme à l'invention met en oeuvre une technique d'amplification génomique desdits virus au cours de l'étape A précitée, celui-ci comprend :
(1) comme étape A, une étape d'enrichissement en séquence(s) cible(s) par :
   (a) mise en contact de l'échantillon biologique mis en solution, avec au moins une paire d'amorces appropriées, pour amplifier au moins un fragment de ladite séquence d'acide nucléique cible, lesdites amorces s'hybridant à ladite séquence cible et permettant d'obtenir une solution d'enrichissement ; et
   (b) au moins une dilution appropriée de la solution d'amplification d'enrichissement obtenue en (a) ;
(2) et une étape B de détection des séquences cibles amplifiées obtenues, par :
   (c) mise en contact d'une fraction de la solution d'enrichissement obtenue en (b) avec au moins une paire d'amorces dont au moins l'une des séquences est incluse dans la séquence cible amplifiée en (a) ; et
   (d) détection des copies d'acide nucléique cible double brin obtenues en (c), par tout moyen approprié, lequel procédé est caractérisé en ce qu'on met en oeuvre, pour l'étape (a), au moins un oligonucléotide conforme à l'invention, apparié avec un oligonucléotide choisi dans le groupe qui comprend les oligonucléotides conformes à l'invention et tout autre oligonucléotide apte à s'hybrider avec lesdites séquences cibles et pour l'étape (c), une paire d'oligonucléotides (amorces) choisis dans le groupe qui comprend les oligonucléotides conformes à l'invention dont la séquence est homologue ou complémentaire de la séquence cible amplifiée en (a) et tout autre oligonucléotide apte à s'hybrider avec les séquences cibles obtenues en (a).

Selon un mode de mise en oeuvre avantageux dudit procédé, les paires d'amorces sont sélectionnées, de préférence, parmi les paires suivantes :
. paire A : oligonucléotide (1) et oligonucléotide (4),
. paire B : oligonucléotide (5) et oligonucléotide (8),
. paire C : oligonucléotide (9) et oligonucléotide (12),
. paire D : oligonucléotide (13) et oligonucléotide (16),
. paire E : oligonucléotide (2) et oligonucléotide (3),
. paire F : oligonucléotide (6) et oligonucléotide (7),
. paire G : oligonucléotide (10) et oligonucléotide (11),
. paire H : oligonucléotide (14) et oligonucléotide (15),
. paire I : oligonucléotide (17) et oligonucléotide (20),de formule 5' TTT GGT CCC TGT CTT ATG TCC AAA ATG C (SEQUENCE ID N°20)
. paire J : oligonucléotide (18) et oligonucléotide (19).

La paire d'amorces A permet d'obtenir une séquence cible d'HIV-1 située sur le gène *tat*, d'une longueur de 171 bases, délimitée par les nucléotides 5419-5500 du génome du virus HIV-1.

La paire d'amorces B permet d'obtenir une séquence cible d'HIV-1 située sur le gène *pol*, d'une longueur de 559 bases, délimitée par les nucléotides 3208 et 3766 du génome du virus HIV-1.

La paire d'amorces C permet d'obtenir une séquence cible d'HIV-2 située sur le gène *tat*, d'une longueur de 197 bases, délimitée par les nucléotides 8311 et 8507 du génome du virus HIV-2.

La paire d'amorces D permet d'obtenir une séquence cible d'HIV-2 située sur le gène *pol*, d'une longueur de 581 bases, délimitée par les nucléotides 3141-3720 du génome du virus HIV-2.

La paire d'amorces E permet d'obtenir une séquence cible d'HIV-1 située sur le gène *tat*, d'une longueur de 114 bases, délimitée par les nucléotides 5447-5560 du génome du virus HIV-1.

La paire d'amorces F permet d'obtenir une séquence cible d'HIV-1 située sur le gène *pol*, d'une longueur de 240 bases, délimitée par les nucléotides 3436 et 3675 du génome du virus HIV-1.

La paire d'amorces G permet d'obtenir une séquence cible d'HIV-2 située sur le gène *tat*, d'une longueur de 91 bases, délimitée par les nucléotides 8363 et 8453 du génome du virus HIV-2.

La paire d'amorces H permet d'obtenir une séquence cible d'HIV-2 située sur le gène *pol*, d'une longueur de 430 bases, délimitée par les nucléotides 3241-3678 du génome du virus HIV-2.

La paire d'amorces I permet d'obtenir une séquence cible HIV-1 située sur le gène *gag*, d'une longueur de 323 bases, délimitée par les nucléotides 882 et 1204 sur le génome du virus HIV-1.

La paire d'amorces J permet d'obtenir une séquence cible HIV-1 située sur le gène *gag*, d'une longueur de 126 bases, et délimitée par les nucléotides 949 et 1074 sur le génome du virus HIV-1.

Selon une disposition avantageuse de ce mode de réalisation, les paires d'amorces de l'étape (a) et les paires d'amorces de l'étape (c) sont de préférence associées selon les groupements suivants :
groupe 1 : paire A - paire E
groupe 2 : paire B - paire F
groupe 3 : paire C - paire G
groupe 4 : paire D - paire H
groupe 5 : paire I - paire J

Egalement conformément à l'invention, les paires d'amorces de l'étape (c) sont des paires : amorce modifiée par un système de capture-amorce modifiée par un système de détection.

On entend par amorce de capture (Ac), une séquence d'acide nucléique simple brin modifiée notamment par au moins une moitié de paire d'affinité et qui s'hybride à une séquence cible ; par exemple, à une extrémité de la chaîne oligonucléotidique une ou plusieurs biotines peuvent être fixées. L'amorce de capture peut également être un oligonucléotide branché.

On entend par amorce de détection (Ad), une séquence d'acide nucléique simple brin modifiée par un système de détection et qui s'hybride à une séquence cible ; par exemple, l'oligonucléotide peut être marqué en son extrémité 5′ par du phosphore 32 et/ou de l'iode 125.

Ce procédé de détection a été dénommé "AMPLICIS"® par les Inventeurs.

La présente invention a de plus pour objet un kit de détection d'un virus HIV, caractérisé en ce qu'il comprend outre les tampons et réactifs appropriés à l'hybridation, à la détection, et éventuellement à l'amplification, au moins un réactif conforme à l'invention.

Outre les dispositions qui précèdent, l'invention comprend encore d'autres dispositions qui ressortiront de la description qui va suivre, qui se réfère à des exemples de mise en oeuvre du procédé objet de la présente invention.

Il doit être bien entendu, toutefois, que ces exemples sont donnés uniquement à titre d'illustration de l'objet de l'invention, dont ils ne constituent en aucune manière une limitation.

### Exemple 1: Détection de HIV-1 tat dans des prélèvements sanguins.

### 1) Protocole opératoire

Les échantillons de sang total sont lysés selon le protocole préconisé par R. HIGUCHI (PCR Technology 1989, H. ERLICH Ed, p. 36, Stockton Press, NY).

25 µl de lysats sanguins sont amplifiés dans un volume de 100 µl de tampon (KCl 50mM, Tris HCl pH 8,3 10mM, MgCl₂ 2,5mM, gélatine 0,1 mg/ml, nonidet NP40 0,45 %, Tween® 20 0,45 %), en présence de 20 pmoles de chacune des amorces externes et de 2,5 unités de Taq DNA polymérase. On effectue une première dénaturation à 94°C pendant 10 minutes, puis 35 cycles d'amplification (dénaturation 94°C 1 minute, hybridation 55°C 1 minute et élongation 72°C 2 minutes). Une dernière incubation de 8 minutes à 72°C permet de terminer les élongations.

Après dilution au 1/200, une partie aliquote (2 µl) de chaque amplification primaire diluée est prélevée et réamplifiée dans un volume total de 25 µl contenant 2,5 pmoles de chaque amorce interne modifiée (l'une avec un résidu biotine, la deuxième avec un iode radioactif ¹²⁵I). L'amplification de détection se poursuit sur 25 cycles (dénaturation 94°C 1 minute, hybridation 55°C 1 minute, élongation 72°C 1 minute). Le produit d'amplification final est fixé par le résidu biotine sur un tube préalablement recouvert avec de l'avidine, et après élimination des amorces en excès par lavages, le tube est compté dans un compteur γ.

### 2) Résultats

- Le tableau I suivant montre une première série de résultats obtenus avec huit lysats sanguins et quatre témoins négatifs.

**TABLEAU I**

| Activité totale par tube : 150 000 cpm | | |
|---|---|---|
| témoin | 1 | 7092 |
| échantillon | 1 | 30267 |
| | 2 | 33769 |
| | 3 | 221 |
| | 4 | 36154 |
| témoin | 2 | 612 |
| échantillon | 5 | 15190 |
| | 6 | 13574 |
| | 7 | 15360 |
| | 8 | 34311 |
| témoin | 3 | 1205 |
| | 4 | 3627 |

Ces résultats ont été comparés aux analyses sur gel et hybridations obtenues à partir des amplifications primaires. On note une très bonne corrélation entre les deux types de détection.

Les échantillons 1, 2, 4, 5, 6, 7 et 8 ont montré bande d'amplification spécifique sur gel après amplification primaire. L'échantillon 3 et les témoins étaient négatifs. Ces résultats se retrouvent de manière identique avec le procédé conforme à l'invention.

- Autre série de résultats de détection des séquences *tat* de HIV-1 :

La première PCR est réalisée sur 1 µg d'ADN extrait de cellules contrôle, de lymphocytes ou de monocytes de patients.

La deuxième PCR est réalisée après une dilution au 1/200 du produit d'amplification de la première PCR. L'amplification est mesurée après 16 ou 32 cycles de PCR II.

Les résultats obtenus avec deux patients (patient A : séropositif-asymptomatique ; patient B : séropositif-SIDA-traitement à l'AZT), sont résumés dans le Tableau II ci-après :

**TABLEAU II**

| Fixation (cpm) | | |
|---|---|---|
| Echantillon | 16 cycles | 32 cycles |
| monocytes A | 13 455 | 56 344 |
| lymphocytes A | 56 424 | 65 916 |
| monocytes B | 1 166 | 5 302 |
| lymphocytes B | 64 766 | 70 782 |
| contrôle - ⁽¹⁾ | 1 657 | 1 611 |
| contrôle + ⁽²⁾ | 67 426 | 68 252 |

| | | |
|---|---|---|
| ⁽¹⁾ U937 | | |
| ⁽²⁾ p8T1-LAV | | |

La présence de séquences HIV-1 rares dans les monocytes du patient B peuvent être détectés après 32 cycles d'amplification.

### Exemple 2 : Détection de HIV-1 pol dans des prélèvements sanguins.

Une détection du fragment *pol* est effectuée sur les prélèvements étudiés avec *tat*, selon un protocole identique à celui décrit à l'exemple 1. Seule différence : les amorces spécifiques du fragment *pol.*

Les résultats obtenus sont illustrés dans le tableau III ci-après :

**TABLEAU III**

| Activité totale par tube : 160 000 cpm | | | |
|---|---|---|---|
| T1 | 112 | E5 | 6178 |
| E1 | 1982 | E6 | 112 |
| E2 | 2854 | E7 | 1305 |
| E3 | 10454 | E8 | 11582 |
| E4 | 6986 | T3 | 115 |
| T2 | 124 | T4 | 99 |

Ces résultats montrent aussi une bonne corrélation avec le Southern et l'hybridation.

### Exemple 3 : Comparaison de la méthode de détection avec les réactifs de l'invention avec une procédure PCR standard (Southern blot).

**TABLEAU IV**

| Echantillon | Southern blot | | | | Procédé "AMPLICIS"® | |
|---|---|---|---|---|---|---|
| | amorces de l'Art ant. | | | amorces *tat* de l'invention | 16 cycles | 32 cycles |
| | *gag* | LTR | *env* | | | |

| Contrôle négatif | | | | | | |
|---|---|---|---|---|---|---|
| . lignée U937 | - | - | - | - | - | - |
| . cellules B | - | - | - | - | | |
| Lymphocytes Patient A | + | - | + | + | +++ | +++ |
| Lymphocytes Patient B | + | + | + | + | +++ | +++ |
| Monocytes Patient A | - | - | - | - | + | +++ |
| Monocytes Patient B | + | - | - | - | - | + |

| Contrôle positif | | | | | | |
|---|---|---|---|---|---|---|
| p8T1-LAV | +++ | +++ | +++ | +++ | +++ | +++ |

Ce Tableau IV montre que :
- d'une part les amorces conformes à l'invention présentent une sensibilité supérieure à celle des amorces de l'Art antérieur ;
- d'autre part, l'utilisation des amorces conformes à l'invention, dans un procédé de détection par amplification du type décrit dans la Demande Internationale PCT/FR90/00410, augmente encore la sensibilité de la détection.

### Exemple 4 : Détection simultanée d'HIV-1/HIV-2.

On a recherché la présence des séquences *pol* des virus HIV-1 et HIV-2 comme précisé à l'exemple 2 ci-dessus dans les lignées CEM infectées par les cellules BRU, NDK et ROD.

| | HIV-1 *pol* (cpm) | HIV-2 *pol* (cpm) |
|---|---|---|
| BRU | 7 923 | 115 |
| NKD | 7 900 | 142 |
| ROD | 184 | 28 173 |
| Activité totale | 140 000 | 190 000 |

Le Tableau ci-dessus montre qu'il n'existe pas de réaction croisée entre HIV-1 et HIV-2.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

### LISTE DES SEOUENCES

- SEQUENCE ID N° :: 1 (correspond à la formule (1))
- TYPE DE SEQUENCE :: nucléotide
- LONGUEUR DE LA SEQUENCE :: 28 pb
- NOMBRE DE BRINS :: simple brin
- CONFIGURATION :: linéaire
- TYPE DE MOLECULE :: ADN
- ORIGINE :: séquence homologue du fragment 5419-5446 issu du gène *tat* du virus HIV-1
- PROPRIETES :: réactif de diagnostic (sonde ou amorce) pour la détection d'un rétrovirus de type HIV.

- SEQUENCE ID N° :: 2 (correspond à la formule (2))
- TYPE DE SEQUENCE :: nucléotide
- LONGUEUR DE LA SEQUENCE :: 28 pb
- NOMBRE DE BRINS :: simple brin
- CONFIGURATION :: linéaire
- TYPE DE MOLECULE :: ADN
- ORIGINE :: séquence homologue du fragment 5447-5474 issu du gène *tat* du virus HIV-1
- PROPRIETES :: réactif de diagnostic (sonde ou amorce) pour la détection d'un rétrovirus de type HIV.

- SEQUENCE ID N° :: 3 (correspond à la formule (3))
- TYPE DE SEQUENCE :: nucléotide
- LONGUEUR DE LA SEQUENCE :: 28 pb
- NOMBRE DE BRINS :: simple brin
- CONFIGURATION :: linéaire
- TYPE DE MOLECULE :: ADN
- ORIGINE :: séquence homologue du fragment 5533-5560 issu du gène *tat* du virus HIV-1
- PROPRIETES :: réactif de diagnostic (sonde ou amorce) pour la détection d'un rétrovirus de type HIV.

- SEQUENCE ID N° :: 4 (correspond à la formule (4))
- TYPE DE SEQUENCE :: nucléotide
- LONGUEUR DE LA SEQUENCE :: 28 pb
- NOMBRE DE BRINS :: simple brin
- CONFIGURATION :: linéaire
- TYPE DE MOLECULE :: ADN
- ORIGINE :: séquence homologue du fragment 5563-5590 issu du gène *tat* du virus HIV-1
- PROPRIETES :: réactif de diagnostic (sonde ou amorce) pour la détection d'un rétrovirus de type HIV.

- SEQUENCE ID N° :: 5 (correspond à la formule (5))
- TYPE DE SEQUENCE :: nucléotide
- LONGUEUR DE LA SEQUENCE :: 28 pb
- NOMBRE DE BRINS :: simple brin
- CONFIGURATION :: linéaire
- TYPE DE MOLECULE :: ADN
- ORIGINE :: séquence homologue du fragment 8311-8338 issu du gène *tat* du virus HIV-2
- PROPRIETES :: réactif de diagnostic (sonde ou amorce) pour la détection d'un rétrovirus de type HIV.

- SEQUENCE ID N° :: 6 (correspond à la formule (6))
- TYPE DE SEQUENCE :: nucléotide
- LONGUEUR DE LA SEQUENCE :: 28 pb
- NOMBRE DE BRINS :: simple brin
- CONFIGURATION :: linéaire
- TYPE DE MOLECULE :: ADN
- ORIGINE :: séquence homologue du fragment 8363-8390 issu du gène *tat* du virus HIV-2
- PROPRIETES :: réactif de diagnostic (sonde ou amorce) pour la détection d'un rétrovirus de type HIV.

- SEQUENCE ID N° :: 7 (correspond à la formule (7))
- TYPE DE SEQUENCE :: nucléotide
- LONGUEUR DE LA SEQUENCE :: 28 pb
- NOMBRE DE BRINS :: simple brin
- CONFIGURATION :: linéaire
- TYPE DE MOLECULE :: ADN
- ORIGINE :: séquence homologue du fragment 8426-8453 issu du gène *tat* du virus HIV-2
- PROPRIETES :: réactif de diagnostic (sonde ou amorce) pour la détection d'un rétrovirus de type HIV.

- SEQUENCE ID N° :: 8 (correspond à la formule (8))
- TYPE DE SEQUENCE :: nucléotide
- LONGUEUR DE LA SEQUENCE :: 28 pb
- NOMBRE DE BRINS :: simple brin
- CONFIGURATION :: linéaire
- TYPE DE MOLECULE :: ADN
- ORIGINE :: séquence homologue du fragment 8480-8507 issu du gène *tat* du virus HIV-2
- PROPRIETES :: réactif de diagnostic (sonde ou amorce) pour la détection d'un rétrovirus de type HIV.

- SEQUENCE ID N° :: 9 (correspond à la formule (9))
- TYPE DE SEQUENCE :: nucléotide
- LONGUEUR DE LA SEQUENCE :: 28 pb
- NOMBRE DE BRINS :: simple brin
- CONFIGURATION :: linéaire
- TYPE DE MOLECULE :: ADN
- ORIGINE :: séquence homologue du fragment 3208-3235 issu du gène *pol* du virus HIV-1
- PROPRIETES :: réactif de diagnostic (sonde ou amorce) pour la détection d'un rétrovirus de type HIV.

- SEQUENCE ID N° :: 10 (correspond à la formule (10))
- TYPE DE SEQUENCE :: nucléotide
- LONGUEUR DE LA SEQUENCE :: 28 pb
- NOMBRE DE BRINS :: simple brin
- CONFIGURATION :: linéaire
- TYPE DE MOLECULE :: ADN
- ORIGINE :: séquence homologue du fragment 3436-3463 issu du gène *pol* du virus HIV-1
- PROPRIETES :: réactif de diagnostic (sonde ou amorce) pour la détection d'un rétrovirus de type HIV.

- SEQUENCE ID N° :: 11 (correspond à la formule (11))
- TYPE DE SEQUENCE :: nucléotide
- LONGUEUR DE LA SEQUENCE :: 29 pb
- NOMBRE DE BRINS :: simple brin
- CONFIGURATION :: linéaire
- TYPE DE MOLECULE :: ADN
- ORIGINE :: séquence homologue du fragment 3647-3675 issu du gène *pol* du virus HIV-1
- PROPRIETES :: réactif de diagnostic (sonde ou amorce) pour la détection d'un rétrovirus de type HIV.

- SEQUENCE ID N° :: 12 (correspond à la formule (12))
- TYPE DE SEQUENCE :: nucléotide
- LONGUEUR DE LA SEQUENCE :: 28 pb
- NOMBRE DE BRINS :: simple brin
- CONFIGURATION :: linéaire
- TYPE DE MOLECULE :: ADN
- ORIGINE :: séquence homologue du fragment 3739-3766 issu du gène *pol* du virus HIV-1
- PROPRIETES :: réactif de diagnostic (sonde ou amorce) pour la détection d'un rétrovirus de type HIV.

- SEQUENCE ID N° :: 13 (correspond à la formule (13))
- TYPE DE SEQUENCE :: nucléotide
- LONGUEUR DE LA SEQUENCE :: 28 pb
- NOMBRE DE BRINS :: simple brin
- CONFIGURATION :: linéaire
- TYPE DE MOLECULE :: ADN
- ORIGINE :: séquence homologue du fragment 3141-3168 issu du gène *pol* du virus HIV-2
- PROPRIETES :: réactif de diagnostic (sonde ou amorce) pour la détection d'un rétrovirus de type HIV.

- SEQUENCE ID N° :: 14 (correspond à la formule (14))
- TYPE DE SEQUENCE :: nucléotide
- LONGUEUR DE LA SEQUENCE :: 28 pb
- NOMBRE DE BRINS :: simple brin
- CONFIGURATION :: linéaire
- TYPE DE MOLECULE :: ADN
- ORIGINE :: séquence homologue du fragment 3241-3268 issu du gène *pol* du virus HIV-2
- PROPRIETES :: réactif de diagnostic (sonde ou amorce) pour la détection d'un rétrovirus de type HIV.

- SEQUENCE ID N° :: 15 (correspond à la formule (15))
- TYPE DE SEQUENCE :: nucléotide
- LONGUEUR DE LA SEQUENCE :: 28 pb
- NOMBRE DE BRINS :: simple brin
- CONFIGURATION :: linéaire
- TYPE DE MOLECULE :: ADN
- ORIGINE :: séquence homologue du fragment 3651-3678 issu du gène *pol* du virus HIV-2
- PROPRIETES :: réactif de diagnostic (sonde ou amorce) pour la détection d'un rétrovirus de type HIV.

- SEQUENCE ID N° :: 16 (correspond à la formule (16))
- TYPE DE SEQUENCE :: nucléotide
- LONGUEUR DE LA SEQUENCE :: 28 pb
- NOMBRE DE BRINS :: simple brin
- CONFIGURATION :: linéaire
- TYPE DE MOLECULE :: ADN
- ORIGINE :: séquence homologue du fragment 3693-3720 issu du gène *pol* du virus HIV-2
- PROPRIETES :: réactif de diagnostic (sonde ou amorce) pour la détection d'un rétrovirus de type HIV.

- SEQUENCE ID N° :: 17 (correspond à la formule (17))
- TYPE DE SEQUENCE :: nucléotide
- LONGUEUR DE LA SEQUENCE :: 28 pb
- NOMBRE DE BRINS :: simple brin
- CONFIGURATION :: linéaire
- TYPE DE MOLECULE :: ADN
- ORIGINE :: séquence homologue du fragment 882-909 issu du gène *gag* du virus HIV-1
- PROPRIETES :: réactif de diagnostic (sonde ou amorce) pour la détection d'un rétrovirus de type HIV.

- SEQUENCE ID N° :: 18 (correspond à la formule (18))
- TYPE DE SEQUENCE :: nucléotide
- LONGUEUR DE LA SEQUENCE :: 28 pb
- NOMBRE DE BRINS :: simple brin
- CONFIGURATION :: linéaire
- TYPE DE MOLECULE :: ADN
- ORIGINE :: séquence homologue du fragment 949-976 issu du gène *gag* du virus HIV-1
- PROPRIETES :: réactif de diagnostic (sonde ou amorce) pour la détection d'un rétrovirus de type HIV.

- SEQUENCE ID N° :: 19 (correspond à la formule (19))
- TYPE DE SEQUENCE :: nucléotide
- LONGUEUR DE LA SEQUENCE :: 28 pb
- NOMBRE DE BRINS :: simple brin
- CONFIGURATION :: linéaire
- TYPE DE MOLECULE :: ADN
- ORIGINE :: séquence homologue du fragment 1047-1074 issu du gène *gag* du virus HIV-1
- PROPRIETES :: réactif de diagnostic (sonde ou amorce) pour la détection d'un rétrovirus de type HIV.

- SEQUENCE ID N° :: 20 (correspond à la formule (20))
- TYPE DE SEQUENCE :: nucléotide
- LONGUEUR DE LA SEQUENCE :: 28 pb
- NOMBRE DE BRINS :: simple brin
- CONFIGURATION :: linéaire
- TYPE DE MOLECULE :: ADN
- ORIGINE :: séquence homologue du fragment 1177-1204 issu du gène *gag* du virus HIV-1
- PROPRIETES :: réactif de diagnostic (sonde ou amorce) pour la détection d'un rétrovirus de type HIV.

## Revendications (Revendications pour l'(les) Etat(s) contractant(s) suivant(s): BE, CH, LI, DE, FR, GB, IT)

1. Oligonucléotides, comprenant environ 25 à 35 nucléotides, pour le diagnostic différentiel des virus HIV-1 et HIV-2, caractérisés en ce qu'ils sont sélectionnés parmi au moins l'une des séquences suivantes :
a) les séquences homologues issues du gène *tat* des HIV-1 et plus particulièrement des virus HIV-1 BRU, HIV-1 ARV, HIV-1 HXB2, HIV-1 BH5, HIV-1 ELI, HIV-1 NDK, HIV-1 MAL, HIV-1 SF2 et les séquences complémentaires de ces séquences, qui correspondent aux positions 5419-5446, 5447-5474, 5533-5560, 5563-5590 du génome du virus HIV-1 BRU ;
b) les séquences homologues issues du gène *tat* des HIV-2 et les séquences complémentaires de ces séquences, qui correspondent aux positions 8311-8338, 8363-8390, 8426-8453, 8480-8507 du génome du virus HIV-2 ROD ;
c) les séquences homologues issues du gène *pol* des HIV-1 et plus particulièrement des virus HIV-1 BRU, HIV-1 ARV, HIV-1 HXB2, HIV-1 BH5, HIV-1 ELI, HIV-1 NDK, HIV-1 MAL, HIV-1 SF2 et les séquences complémentaires de ces séquences, qui correspondent aux positions 3208-3235, 3436-3463, 3647-3675, 3739-3766 du génome du virus HIV-1 BRU ;
d) les séquences homologues issues du gène *pol* des HIV-2 et les séquences complémentaires de ces séquences, qui correspondent aux positions 3141-3168, 3241-3268, 3651-3678, 3693-3720 du génome du virus HIV-2 ROD ; et
e) les séquences homologues issues du gène *gag* dans HIV-1 et plus particulièrement des virus HIV-1 BRU, HIV-1 ARV, HIV-1 HXB2, HIV-1 BH5, HIV-1 ELI, HIV-1 NDK, HIV-1 MAL, HIV-1 SF2 et les séquences complémentaires de ces séquences et qui correspondent aux positions 882-909, 949-976 et 1047-1074 du génome du virus HIV-1 BRU.

2. Oligonucléotides selon la revendication 1, caractérisés en ce qu'ils répondent à l'une quelconque des formules suivantes :

(1) 5' CAG TAG ATC CTA GAC TAG AGC CCT GGA A (SEQUENCE ID N° 1)

(2) 5' GCA TCC AGG AAG TCA GCC TAG GAC TGC T (SEQUENCE ID N° 2)

(3) 5' TTC CTG CCA TAG GAG ATG CCT AAG GCT T (SEQUENCE ID N° 3)

(4) 5' TGA GGA GGT CTT CGT CGC TGT CTC CGC T (SEQUENCE ID N° 4)

(5) 5' TGC CCA CAC TAA TGA TGT AAA ACA ATT A (SEQUENCE ID N° 5)

(6) 5' AGG· AGC AGA AAC TTT CTA TGT AGA TGG G (SEQUENCE ID N° 6)

(7) 5' ATT CAC TTT TAT CTG GTT GTG CTT GAA T (SEQUENCE ID N° 7)

(8) 5' ATT TCC TCC AAT TCC TTT GTG TGC TGG T (SEQUENCE ID N° 8)

(9) 5' ATA TCC ACA AGG ACC GGG GAC AGC CAG C (SEQUENCE ID N° 9)

(10) 5' TGG AAG CAA CGG TGG AGA CAG ATA CTG G (SEQUENCE ID N° 10)

(11) 5' TAG TCT GGT CAA GAG GCG AAT CAG CTG G (SEQUENCE ID N° 11)

(12) 5' GAT GAG TTG GAG GGT CAG GAA GCT CCT G (SEQUENCE ID N° 12)

(13) 5' TCA ATG ACA TCC AGA AGC TAG TGG GTG T (SEQUENCE ID N° 13)

(14) 5' GAC ACT CAC AGA AGA AGT ACA GTG GAC A (SEQUENCE ID N° 14)

(15) 5' ATA GGA TCC CCT ACC AGG TTA AAC GCT A (SEQUENCE ID N° 15)

(16) 5' CTA TTG CAG GAT CCA TCT GTG TAG AAG G (SEQUENCE ID N° 16)

(17) 5' GAT TTA AAC ACC ATG CTA AAC ACA GTG G (SEQUENCE ID N° 17)

(18) 5' TCA ATG AGG AAG CTG CAG AAT GGG ATA G (SEQUENCE ID N° 18)

(19) 5' TTT GTT CCT GAA GGG TAC TAG TAG TTC C (SEQUENCE ID N° 19)

3. Réactif de diagnostic pour la détection d'au moins un rétrovirus de type HIV (HIV-1 et/ou HIV-2), caractérisé en ce qu'il comprend au moins un oligonucléotide selon la revendication 1 ou la revendication 2, éventuellement marqué de manière appropriée.

4. Réactif selon la revendication 3, caractérisé en ce qu'il correspond à des paires d'amorces pour la synthèse d'un fragment nucléique d'HIV.

5. Réactif selon la revendication 4, caractérisé en ce qu'il est notamment constitué par l'une des paires d'amorces suivantes :
. paire A : oligonucléotide (1) et oligonucléotide (4),
. paire B : oligonucléotide (5) et oligonucléotide (8),
. paire C : oligonucléotide (9) et oligonucléotide (12),
. paire D : oligonucléotide (13) et oligonucléotide (16),
. paire E : oligonucléotide (2) et oligonucléotide (3),
. paire F : oligonucléotide (6) et oligonucléotide (7),
. paire G : oligonucléotide (10) et oligonucléotide (11),
. paire H : oligonucléotide (14) et oligonucléotide (15),
. paire I : oligonucléotide (17) et oligonucléotide (20), de formule 5' TTT GGT CCC TGT CTT ATG TCC AAA ATG C (SEQUENCE ID N°20)
. paire J : oligonucléotide (18) et oligonucléotide (19).

6. Réactif selon la revendication 3, caractérisé en ce qu'il correspond à une sonde de détection ou de révélation d'un fragment nucléique d'HIV et est marqué de manière convenable.

7. Procédé de détection d'un virus HIV par la détection de sa séquence d'acide nucléique dans un échantillon biologique, caractérisé en ce qu'il comprend, après une mise en solution appropriée de l'échantillon biologique pour extraire l'acide nucléique :
A. une étape dans laquelle on met en contact l'échantillon biologique avec au moins un réactif de diagnostic choisi dans le groupe qui comprend les réactifs selon l'une quelconque des revendications 3 à 6 et tout autre réactif apte à s'hybrider avec la séquence à détecter, et
B. une étape dans laquelle on détecte par tout moyen approprié le/les produits résultant de l'interaction séquence nucléotidique d'HIV éventuellement présente-réactif de diagnostic.

8. Procédé selon la revendication 7, caractérisé en ce que le réactif de diagnostic de l'étape A est choisi dans le groupe qui comprend les paires d'amorces dont au moins l'une d'entre elles est un oligonucléotide selon la revendication 1 ou la revendication 2 et les paires d'amorces convenables à l'amplification de la séquence nucléotidique d'HIV, lequel procédé permet l'obtention du produit d'amplification de la séquence nucléotidique à détecter.

9. Procédé selon la revendication 8, caractérisé en ce que les produits d'amplification obtenus en A sont détectés par hybridation entre lesdits produits d'amplification et un réactif de diagnostic selon l'une quelconque des revendications 3 à 6, marqué de manière approprié.

10. Procédé selon la revendication 7, caractérisé en ce que le réactif de diagnostic de l'étape A est une sonde de détection ou de révélation selon la revendication 3 ou la revendication 6, lequel procédé permet l'obtention d'hybrides entre la séquence nucléotidique à détecter et ladite sonde.

11. Procédé selon la revendication 7, mettant en oeuvre une technique d'amplification génomique desdits virus au cours de l'étape A précitée, lequel procédé comprend, après la mise en solution appropriée de l'échantillon biologique pour extraire l'acide nucléique, la détection de la séquence d'acide nucléique du HIV recherché à l'aide des étapes suivantes :
(1) comme étape A, une étape d'enrichissement en séquence(s) cible(s) par :
(a) mise en contact de l'échantillon biologique mis en solution, avec au moins une paire d'amorces appropriées, pour amplifier au moins un fragment de ladite séquence d'acide nucléique cible, lesdites amorces s'hybridant à ladite séquence cible et permettant d'obtenir une solution d'enrichissement ; et
(b) au moins une dilution appropriée de la solution d'amplification d'enrichissement obtenue en (a) ;
(2) et une étape B de détection des séquences cibles amplifiées obtenues, par :
(c) mise en contact d'une fraction de la solution d'enrichissement obtenue en (b) avec au moins une paire d'amorces dont au moins l'une des séquences est incluse dans la séquence cible amplifiée en (a) ; et
(d) détection des copies d'acide nucléique cible double brin obtenues en (c), par tout moyen approprié,
lequel procédé est caractérisé en ce qu'on met en oeuvre, pour l'étape (a), au moins un oligonucléotide selon la revendication 1 ou la revendication 2, apparié avec un oligonucléotide choisi dans le groupe qui comprend les oligonucléotides selon la revendication 1 et la revendication 2 et tout autre oligonucléotide apte à s'hybrider avec lesdites séquences cibles et pour l'étape (c), une paire d'oligonucléotides (amorces) choisis dans le groupe qui comprend les réactifs selon l'une quelconque des revendications 3 à 5 dont la séquence est homologue ou complémentaire de la séquence cible amplifiée en (a) et tout autre oligonucléotide apte à s'hybrider avec les séquences cibles obtenues en (a).

12. Procédé selon la revendication 11, caractérisé en ce que les paires d'amorces sont sélectionnées, de préférence, parmi les paires suivantes :
. paire A : oligonucléotide (1) et oligonucléotide (4),
. paire B : oligonucléotide (5) et oligonucléotide (8),
. paire C : oligonucléotide (9) et oligonucléotide (12),
. paire D : oligonucléotide (13) et oligonucléotide (16),
. paire E : oligonucléotide (2) et oligonucléotide (3),
. paire F : oligonucléotide (6) et oligonucléotide (7),
. paire G : oligonucléotide (10) et oligonucléotide (11),
. paire H : oligonucléotide (14) et oligonucléotide (15),
. paire I : oligonucléotide (17) et oligonucléotide (20),
de formule 5' TTT GGT CCC TGT CTT ATG TCC AAA ATG C (SEQUENCE ID N°20)
. paire J : oligonucléotide (18) et oligonucléotide (19).

13. Procédé selon la revendication 11 ou la revendication 12, caractérisé en ce que les paires d'amorces de l'étape (a) et les paires d'amorces de l'étape (c) sont de préférence associées selon les groupements suivants :
groupe 1 : paire A - paire E
groupe 2 : paire B - paire F
groupe 3 : paire C - paire G
groupe 4 : paire D - paire H
groupe 5 : paire I - paire J

14. Procédé selon l'une quelconque des revendications 11 à 13, caractérisé en ce que les paires d'amorces de l'étape (c) sont des paires : amorce modifiée par un système de capture-amorce modifiée par un système de détection.

15. Kit de détection d'un virus HIV, caractérisé en ce qu'il comprend outre les tampons et réactifs appropriés à l'hybridation, à la détection, et éventuellement à l'amplification, au moins un réactif selon l'une quelconque des revendications 3 à 6.

## Revendications (Revendications pour l'(les) Etat(s) contractant(s) suivant(s): ES)

1. Procédé d'obtention d'un oligonucléotide, comprenant environ 25 à 35 nucléotides, pour le diagnostic différentiel des virus HIV-1 et HIV-2, caractérisé en ce que l'on prépare et l'on sélectionne au moins l'une des séquences suivantes :
a) les séquences homologues issues du gène *tat* des HIV-1 et plus particulièrement des virus HIV-1 BRU, HIV-1 ARV, HIV-1 HXB2, HIV-1 BH5, HIV-1 ELI, HIV-1 NDK, HIV-1 MAL, HIV-1 SF2 et les séquences complémentaires de ces séquences, qui correspondent aux positions 5419-5446, 5447-5474, 5533-5560, 5563-5590 du génome du virus HIV-1 BRU ;
b) les séquences homologues issues du gène *tat* des HIV-2 et les séquences complémentaires de ces séquences, qui correspondent aux positions 8311-8338, 8363-8390, 8426-8453, 8480-8507 du génome du virus HIV-2 ROD ;
c) les séquences homologues issues du gène *pol* des HIV-1 et plus particulièrement des virus HIV-1 BRU, HIV-1 ARV, HIV-1 HXB2, HIV-1 BH5, HIV-1 ELI, HIV-1 NDK, HIV-1 MAL, HIV-1 SF2 et les séquences complémentaires de ces séquences, qui correspondent aux positions 3208-3235, 3436-3463, 3647-3675, 3739-3766 du génome du virus HIV-1 BRU ;
d) les séquences homologues issues du gène *pol* des HIV-2 et les séquences complémentaires de ces séquences, qui correspondent aux positions 3141-3168, 3241-3268, 3651-3678, 3693-3720 du génome du virus HIV-2 ROD ; et
e) les séquences homologues issues du gène *gag* dans HIV-1 et plus particulièrement des virus HIV-1 BRU, HIV-1 ARV, HIV-1 HXB2, HIV-1 BH5, HIV-1 ELI, HIV-1 NDK, HIV-1 MAL, HIV-1 SF2 et les séquences complémentaires de ces séquences et qui correspondent aux positions 882-909, 949-976 et 1047-1074 du génome du virus HIV-1 BRU.

2. Procédé d'obtention d'un oligonucléotide selon la revendication 1, caractérisé en ce que l'on prépare un oligonucléotide qui répond à l'une quelconque des formules suivantes :

(1) 5' CAG TAG ATC CTA GAC TAG AGC CCT GGA A
(séquence ID N° 1)

(2) 5' GCA TCC AGG AAG TCA GCC TAG GAC TGC T
(séquence ID N° 2)

(3) 5' TTC CTG CCA TAG GAG ATG CCT AAG GCT T
(séquence ID N° 3)

(4) 5' TGA GGA GGT CTT CGT CGC TGT CTC CGC T
(séquence ID N° 4)

(5) 5' TGC CCA CAC TAA TGA TGT AAA ACA ATT A
(séquence ID N° 5)

(6) 5' AGG AGC AGA AAC TTT CTA TGT AGA TGG G
(séquence ID N° 6)

(7) 5' ATT CAC TTT TAT CTG GTT GTG CTT GAA T
(séquence ID N° 7)

(8) 5' ATT TCC TCC AAT TCC TTT GTG TGC TGG T
(séquence ID N° 8)

(9) 5' ATA TCC ACA AGG ACC GGG GAC AGC CAG C
(séquence ID N° 9)

(10) 5' TGG AAG CAA CGG TGG AGA CAG ATA CTG G
(séquence ID N° 10)

(11) 5' TAG TCT GGT CAA GAG GCG AAT CAG CTG G
(séquence ID N° 11)

(12) 5' GAT GAG TTG GAG GGT CAG GAA GCT CCT G
(séquence ID N° 12)

(13) 5' TCA ATG ACA TCC AGA AGC TAG TGG GTG T
(séquence ID N° 13)

(14) 5' GAC ACT CAC AGA AGA AGT ACA GTG GAC A
(séquence ID N° 14)

(15) 5' ATA GGA TCC CCT ACC AGG TTA AAC GCT A
(séquence ID N° 15)

(16) 5' CTA TTG CAG GAT CCA TCT GTG TAG AAG G
(séquence ID N° 16)

(17) 5' GAT TTA AAC ACC ATG CTA AAC ACA GTG G
(séquence ID N° 17)

(18) 5' TCA ATG AGG AAG CTG CAG AAT GGG ATA G
(séquence ID N° 18)

(19) 5' TTT GTT CCT GAA GGG TAC TAG TAG TTC C
(séquence ID N° 19)

3. Procédé d'obtention d'un réactif de diagnostic pour la détection d'au moins un rétrovirus de type HIV (HIV-1 et/ou HIV-2), caractérisé en ce que l'on prépare ledit réactif à partir d'au moins un oligonucléotide selon la revendication 1 ou la revendication 2, et en ce que éventuellement on marque ledit oligonucléotide de manière appropriée.

4. Procédé d'obtention d'un réactif selon la revendication 3, caractérisé en ce que l'on prépare ledit réactif à partir de paires d'amorces pour la synthèse d'un fragment nucléique d'HIV.

5. Procédé d'obtention d'un réactif selon la revendication 4, caractérisé en ce que les paires d'amorces sont sélectionnées parmi les paires d'amorces suivantes :
. paire A : oligonucléotide (1) et oligonucléotide (4),
. paire B : oligonucléotide (5) et oligonucléotide (8),
. paire C : oligonucléotide (9) et oligonucléotide (12),
. paire D : oligonucléotide (13) et oligonucléotide (16),
. paire E : oligonucléotide (2) et oligonucléotide (3),
. paire F : oligonucléotide (6) et oligonucléotide (7),
. paire G : oligonucléotide (10) et oligonucléotide (11),
. paire H : oligonucléotide (14) et oligonucléotide (15),
. paire I : oligonucléotide (17) et oligonucléotide (20), de formule 5' TTT GGT CCC TGT CTT ATG TCC AAA ATG C (séquence ID N° 20)
. paire J : oligonucléotide (18) et oligonucléotide (19).

6. Procédé d'obtention d'un réactif selon la revendication 3, caractérisé en ce que l'on prépare ledit réactif à partir d'une sonde de détection ou de révélation d'un fragment nucléique d'HIV et que l'on marque ladite sonde de manière convenable.

7. Procédé de détection d'un virus HIV par la détection de sa séquence d'acide nucléique dans un échantillon biologique, caractérisé en ce qu'il comprend, après une mise en solution appropriée de l'échantillon biologique pour extraire l'acide nucléique :
A. une étape dans laquelle on met en contact l'échantillon biologique avec au moins un réactif de diagnostic choisi dans le groupe qui comprend les réactifs selon l'une quelconque des revendications 3 à 6 et tout autre réactif apte à s'hybrider avec la séquence à détecter, et
B. une étape dans laquelle on détecte par tout moyen approprié le/les produits résultant de l'interaction séquence nucléotidique d'HIV éventuellement présente-réactif de diagnostic.

8. Procédé selon la revendication 7, caractérisé en ce que le réactif de diagnostic de l'étape A est choisi dans le groupe qui comprend les paires d'amorces dont au moins l'une d'entre elles est un oligonucléotide selon la revendication 1 ou la revendication 2 et les paires d'amorces convenables à l'amplification de la séquence nucléotidique d'HIV, lequel procédé permet l'obtention du produit d'amplification de la séquence nucléotidique à détecter.

9. Procédé selon la revendication 8, caractérisé en ce que les produits d'amplification obtenus en A sont détectés par hybridation entre lesdits produits d'amplification et un réactif de diagnostic selon l'une quelconque des revendications 3 à 6, marqué de manière approprié.

10. Procédé selon la revendication 7, caractérisé en ce que le réactif de diagnostic de l'étape A est une sonde de détection ou de révélation selon la revendication 3 ou la revendication 6, lequel procédé permet l'obtention d'hybrides entre la séquence nucléotidique à détecter et ladite sonde.

11. Procédé selon la revendication 7, mettant en oeuvre une technique d'amplification génomique desdits virus au cours de l'étape A précitée, lequel procédé comprend, après la mise en solution appropriée de l'échantillon biologique pour extraire l'acide nucléique, la détection de la séquence d'acide nucléique du HIV recherché à l'aide des étapes suivantes :
(1) comme étape A, une étape d'enrichissement en séquence(s) cible(s) par :
(a) mise en contact de l'échantillon biologique mis en solution, avec au moins une paire d'amorces appropriées, pour amplifier au moins un fragment de ladite séquence d'acide nucléique cible, lesdites amorces s'hybridant à ladite séquence cible et permettant d'obtenir une solution d'enrichissement ; et
(b) au moins une dilution appropriée de la solution d'amplification d'enrichissement obtenue en (a) ;
(2) et une étape B de détection des séquences cibles amplifiées obtenues, par :
(c) mise en contact d'une fraction de la solution d'enrichissement obtenue en (b) avec au moins une paire d'amorces dont au moins l'une des séquences est incluse dans la séquence cible amplifiée en (a) ; et
(d) détection des copies d'acide nucléique cible double brin obtenues en (c), par tout moyen approprié,
lequel procédé est caractérisé en ce qu'on met en oeuvre, pour l'étape (a), au moins un oligonucléotide selon la revendication 1 ou la revendication 2, apparié avec un oligonucléotide choisi dans le groupe qui comprend les oligonucléotides selon la revendication 1 et la revendication 2 et tout autre oligonucléotide apte à s'hybrider avec lesdites séquences cibles et pour l'étape (c), une paire d'oligonucléotides (amorces) choisis dans le groupe qui comprend les réactifs selon l'une quelconque des revendications 3 à 5 dont la séquence est homologue ou complémentaire de la séquence cible amplifiée en (a) et tout autre oligonucléotide apte à s'hybrider avec les séquences cibles obtenues en (a).

12. Procédé selon la revendication 11, caractérisé en ce que les paires d'amorces sont sélectionnées, de préférence, parmi les paires suivantes :
. paire A : oligonucléotide (1) et oligonucléotide (4),
. paire B : oligonucléotide (5) et oligonucléotide (8),
. paire C : oligonucléotide (9) et oligonucléotide (12),
. paire D : oligonucléotide (13) et oligonucléotide (16),
. paire E : oligonucléotide (2) et oligonucléotide (3),
. paire F : oligonucléotide (6) et oligonucléotide (7),
. paire G : oligonucléotide (10) et oligonucléotide (11),
. paire H : oligonucléotide (14) et oligonucléotide (15),
. paire I : oligonucléotide (17) et oligonucléotide (20), de formule 5' TTT GGT CCC TGT CTT ATG TCC AAA ATG C (séquence ID N° 20)
. paire J : oligonucléotide (18) et oligonucléotide (19).

13. Procédé selon la revendication 11 ou la revendication 12, caractérisé en ce que les paires d'amorces de l'étape (a) et les paires d'amorces de l'étape (c) sont de préférence associées selon les groupements suivants :
groupe 1 : paire A - paire E
groupe 2 : paire B - paire F
groupe 3 : paire C - paire G
groupe 4 : paire D - paire H
groupe 5 : paire I - paire J

14. Procédé selon l'une quelconque des revendications 11 à 13, caractérisé en ce que les paires d'amorces de l'étape (c) sont des paires : amorce modifiée par un système de capture-amorce modifiée par un système de détection.

15. Procédé de préparation d'un kit de détection d'un virus HIV, caractérisé en ce que l'on associe les tampons et réactifs appropriés à l'hybridation, à la détection, et éventuellement à l'amplification, à au moins un réactif obtenu selon l'une quelconque des revendications 3 à 6.

## Patentansprüche (Patentansprüche für folgende(n) Vertragsstaat(en): BE, CH, LI, DE, FR, GB, IT)

1. Oligonukleotide mit etwa 25 bis 35 Nukleotiden für die Differentialdiagnose der Viren HIV-1 und HIV-2, dadurch **gekennzeichnet,** daß sie aus mindestens einer der folgenden Sequenzen selektioniert sind:
a) den aus dem Gen *tat* der Viren HIV-1 und insbesondere der Viren HIV-1 BRU, HIV-1 ARV, HIV-1 HXB2, HIV-1 BH5, HIV-1 ELI, HIV-1 NDK, HIV-1 MAL, HIV-1 SF2 stammenden homologen Sequenzen und den diesen Sequenzen komplementären Sequenzen, die den Positionen 5419-5446, 5447-5474, 5533-5560, 5563-5590 des Genoms des Virus HIV-1 BRU entsprechen;
b) den aus dem Gen *tat* der Viren HIV-2 stammenden homologen Sequenzen und den diesen Sequenzen komplementären Sequenzen, die den Positionen 8311-8338, 8363-8390, 8426-8453, 8480-8507 des Genoms des Virus HIV-2 ROD entsprechen;
c) den aus dem Gen *pol* der Viren HIV-1 und insbesondere der Viren HIV-1 BRU, HIV-1 ARV, HIV-1 HXB2, HIV-1 BH5, HIV-1 ELI, HIV-1 NDK, HIV-1 MAL, HIV-1 SF2 stammenden homologen Sequenzen und den diesen Sequenzen komplementären Sequenzen, die den Positionen 3208-3235, 3436-3463, 3647-3675, 3739-3766 des Genoms des Virus HIV-1 BRU entsprechen;
d) den aus dem Gen *pol* der Viren HIV-2 stammenden homologen Sequenzen und den diesen Sequenzen komplementären Sequenzen, die den Positionen 3141-3168, 3241-3268, 3651-3678, 3693-3720 des Genoms des Virus HIV-2 ROD entsprechen; und
e) den aus dem Gen *gag* in HIV-1 und insbesondere der Viren HIV-1 BRU, HIV-1 ARV, HIV-1 HXB2, HIV-1 BH5, HIV-1 ELI, HIV-1 NDK, HIV-1 MAL, HIV-1 SF2 stammenden homologen Sequenzen und den diesen Sequenzen komplementären Sequenzen, die den Positionen 882-909, 949-976 und 1047-1074 des Genoms des Virus HIV-1 BRU entsprechen.

2. Oligonukleotide nach Anspruch 1, dadurch **gekennzeichnet,** daß sie einer der folgenden Formeln entsprechen:

(1) 5' CAG TAG ATC CTA GAC TAG AGC CCT GGA A (Sequenz ID Nr. 1)
(2) 5' GCA TCC AGG AAG TCA GCC TAG GAC TGC T (Sequenz ID Nr. 2)
(3) 5' TTC CTG CCA TAG GAG ATG CCT AAG GCT T (Sequenz ID Nr. 3)
(4) 5' TGA GGA GGT CTT CGT CGC TGT CTC CGC T (Sequenz ID Nr. 4)
(5) 5' TGC CCA CAC TAA TGA TGT AAA ACA ATT A (Sequenz ID Nr. 5)
(6) 5' AGG AGC AGA AAC TTT CTA TGT AGA TGG G (Sequenz ID Nr. 6)
(7) 5' ATT CAC TTT TAT CTG GTT GTG CTT GAA T (Sequenz ID Nr. 7)
(8) 5' ATT TCC TCC AAT TCC TTT GTG TGC TGG T (Sequenz ID Nr. 8)
(9) 5' ATA TCC ACA AGG ACC GGG GAC AGC CAG C (Sequenz ID Nr. 9)
(10) 5' TGG AAG CAA CGG TGG AGA CAG ATA CTG G (Sequenz ID Nr. 10)
(11) 5' TAG TCT GGT CAA GAG GCG AAT CAG CTG G (Sequenz ID Nr. 11)
(12) 5' GAT GAG TTG GAG GGT CAG GAA GCT CCT G (Sequenz ID Nr. 12)
(13) 5' TCA ATG ACA TCC AGA AGC TAG TGG GTG T (Sequenz ID Nr. 13)
(14) 5' GAC ACT CAC AGA AGA AGT ACA GTG GAC A (Sequenz ID Nr. 14)
(15) 5' ATA GGA TCC CCT ACC AGG TTA AAC GCT A (Sequenz ID Nr. 15)
(16) 5' CTA TTG CAG GAT CCA TCT GTG TAG AAG G (Sequenz ID Nr. 16)
(17) 5' GAT TTA AAC ACC ATG CTA AAC ACA GTG G (Sequenz ID Nr. 17)
(18) 5' TCA ATG AGG AAG CTG CAG AAT GGG ATA G (Sequenz ID Nr. 18)
(19) 5' TTT GTT CCT GAA GGG TAC TAG TAG TTC C (Sequenz ID Nr. 19)

3. Diagnosereagens für die Detektion mindestens eines Retrovirus vom Typ HIV (HIV-1 und/oder HIV-2), dadurch **gekennzeichnet,** daß es mindestens ein Oligonukleotid nach Anspruch 1 oder Anspruch 2, gegebenenfalls in geeigneter Weise markiert, enthält.

4. Reagens nach Anspruch 3, dadurch **gekennzeichnet,** daß es Starterpaaren für die Synthese eines Nukleinsäurefragments von HIV entspricht.

5. Reagens nach Anspruch 4, dadurch **gekennzeichnet,** daß es insbesondere aus einem der folgenden Starterpaaren besteht:
. Paar A : Oligonukleotid (1) und Oligonukleotid (4),
. Paar B : Oligonukleotid (5) und Oligonukleotid (8),
. Paar C : Oligonukleotid (9) und Oligonukleotid (12),
. Paar D : Oligonukleotid (13) und Oligonukleotid (16),
. Paar E : Oligonukleotid (2) und Oligonukleotid (3),
. Paar F : Oligonukleotid (6) und Oligonukleotid (7),
. Paar G : Oligonukleotid (10) und Oligonukleotid (11),
. Paar H : Oligonukleotid (14) und Oligonukleotid (15),
. Paar I : Oligonukleotid (17) und Oligonukleotid (20), der Formel 5' TTT GGT CCC TGT CTT ATG TCC AAA ATG C (Sequenz ID Nr. 20)
. Paar J : Oligonukleotid (18) und Oligonukleotid (19).

6. Reagens nach Anspruch 3, dadurch **gekennzeichnet,** daß es einer Sonde für die Detektion oder den Nachweis eines Nukleinsäurefragments von HIV entspricht und in geeigneter Weise markiert ist.

7. Verfahren zur Detektion eines HIV-Virus durch Detektion seiner Nukleinsäuresequenz in einer biologischen Probe, dadurch **gekennzeichnet,** daß es nach geeignetem Inlösungbringen der biologischen Probe, um die Nukleinsäure zu extrahieren, umfaßt:
A. eine Stufe, in der man die biologische Probe mit mindestens einem Diagnosereagens in Berührung bringt, das aus der Gruppe ausgewählt ist, die die Reagentien gemäß einem der Ansprüche 3 bis 6 und jedes andere Reagens, das mit der zu detektierenden Sequenz hybridisieren kann, umfaßt, und
B. eine Stufe, in der man mit jedem geeigneten Mittel das/die Produkt/Produkte detektiert, die aus der Wechselwirkung zwischen gegebenenfalls vorhandener Nukleotidsequenz des HIV und dem Diagnosereagens resultieren.

8. Verfahren nach Anspruch 7, dadurch **gekennzeichnet,** daß das Diagnosereagens der Stufe A aus der Gruppe ausgewählt wird, die die Starterpaare, von denen mindestens eines ein Oligonukleotid nach Anspruch 1 oder Anspruch 2 ist und die für die Amplifikation der Nukleotidsequenz des HIV geeigneten Starterpaare umfaßt und das Verfahren ermöglicht, das Amplifikationsprodukt der zu detektierenden Nukleotidsequenz zu erhalten.

9. Verfahren nach Anspruch 8, dadurch **gekennzeichnet**, daß die in A erhaltenen Produkte der Amplifikation durch Hybridisierung dieser Produkte der Amplifikation mit einem Diagnosereagens nach einem der Ansprüche 3 bis 6, in geeigneter Weise markiert, detektiert werden.

10. Verfahren nach Anspruch 7, dadurch **gekennzeichnet,** daß das Diagnosereagens der Stufe A eine Sonde für die Detektion oder den Nachweis nach Anspruch 3 oder Anspruch 6 ist und das Verfahren den Erhalt von Hybriden zwischen der nachzuweisenden Nukleotidsequenz und der genannten Sonde ermöglicht.

11. Verfahren nach Anspruch 7, bei dem eine Technik der genomischen Amplifikation der genannten Viren im Verlauf der vorgenannten Stufe A angewandt wird, wobei das Verfahren nach geeignetem Inlösungbringen der biologischen Probe, um die Nukleinsäure zu extrahieren, die Detektion der Nukleinsäuresequenz des gesuchten HIV mit Hilfe der folgenden Stufen umfaßt:
(1) als Stufe A, eine Stufe der Anreicherung an Zielsequenz(en) durch:
(a) Inkontaktbringen der in Lösung gebrachten biologischen Probe mit mindestens einem Paar geeigneter Starter, um mindestens ein Fragment der genannten Nukleinsäure-Zielsequenz zu amplifizieren, wobei die Starter mit der Zielsequenz hybridisieren und erlauben, eine Anreicherungslösung zu erhalten; und
(b) mindestens eine geeignete Verdünnung der in (a) erhaltenen Amplifikations-Anreicherungslösung;
(2) eine Stufe B der Detektion der erhaltenen amplifizierten Zielsequenzen durch:
(c) Inkontaktbringen einer Fraktion der in (b) erhaltenen Anreicherungslösung mit mindestens einem Starterpaar, von denen mindestens eine der Sequenzen in die in (a) amplifizierte Zielsequenz eingeschlossen ist; und
(d) Detektion der in (c) erhaltenen Kopien doppelsträngiger Zielnukleinsäure mit jedem geeigneten Mittel, dadurch **gekennzeichnet,** daß man für die Stufe (a) mindestens ein Oligonukleotid nach Anspruch 1 oder Anspruch 2 einsetzt, das mit einem Oligonukleotid gepaart ist, ausgewählt aus der Gruppe, umfassend die Oligonukleotide nach Anspruch 1 und Anspruch 2 und jedes andere Oligonukleotid, das mit den genannten Zielsequenzen hybridisieren kann, und daß man für die Stufe (c) ein Paar von Oligonukleotiden (Startern) einsetzt, die aus der Gruppe ausgewählt sind, welche die Reagentien nach einem der Ansprüche 3 bis 5 umfaßt, deren Sequenz der in (a) amplifizierten Zielsequenz homolog oder komplementär ist, sowie jedes andere Oligonukleotid, das mit den in (a) erhaltenen Zielsequenzen hybridisieren kann.

12. Verfahren nach Anspruch 11, dadurch **gekennzeichnet,** daß die Starterpaare unter folgenden Paaren ausgewählt werden:
. Paar A : Oligonukleotid (1) und Oligonukleotid (4),
. Paar B : Oligonukleotid (5) und Oligonukleotid (8),
. Paar C : Oligonukleotid (9) und Oligonukleotid (12),
. Paar D : Oligonukleotid (13) und Oligonukleotid (16),
. Paar E : Oligonukleotid (2) und Oligonukleotid (3),
. Paar F : Oligonukleotid (6) und Oligonukleotid (7),
. Paar G : Oligonukleotid (10) und Oligonukleotid (11),
. Paar H : Oligonukleotid (14) und Oligonukleotid (15),
. Paar I : Oligonukleotid (17) und Oligonukleotid (20), (Sequenz ID Nr. 20) der Formel 5' TTT GGT CCC TGT CTT ATG TCC AAA ATG C
. Paar J : Oligonukleotid (18) und Oligonukleotid (19).

13. Verfahren nach Anspruch 11 oder Anspruch 12, dadurch **gekennzeichnet,** daß die Starterpaare der Stufe (a) und die Starterpaare der Stufe (c) vorzugsweise entsprechend den folgenden Gruppen assoziiert sind:
Gruppe 1 : Paar A - Paar E
Gruppe 2 : Paar B - Paar F
Gruppe 3 : Paar C - Paar G
Gruppe 4 : Paar D - Paar H
Gruppe 5 : Paar I - Paar J

14. Verfahren nach einem der Ansprüche 11 bis 13, dadurch **gekennzeichnet,** daß die Starterpaare der Stufe (c) Paare sind aus Starter, modifiziert mit einem Einfangsystem, und Starter, modifiziert mit einem Detektionssystem.

15. Kit für die Detektion eines HIV-Virus, dadurch **gekennzeichnet,** daß er zusätzlich zu den für die Hybridisierung, die Detektion und gegebenenfalls die Amplifikation geeigneten Puffern und Reagentien mindestens ein Reagens nach einem der Ansprüche 3 bis 6 enthält.

## Patentansprüche (Patentansprüche für folgende(n) Vertragsstaat(en): ES)

1. Verfahren zum Erhalt eines Oligonukleotids mit etwa 25 bis 35 Nukleotiden für die Differentialdiagnose der Viren HIV-1 und HIV-2, dadurch **gekennzeichnet,** daß man mindestens eine der folgenden Sequenzen herstellt und selektioniert:
a) die aus dem Gen *tat* der Viren HIV-1 und insbesondere der Viren HIV-1 BRU, HIV-1 ARV, HIV-1 HXB2, HIV-1 BH5, HIV-1 ELI, HIV-1 NDK, HIV-1 MAL, HIV-1 SF2 stammenden homologen Sequenzen und die diesen Sequenzen komplementären Sequenzen, die den Positionen 5419-5446, 5447-5474, 5533-5560, 5563-5590 des Genoms des Virus HIV-1 BRU entsprechen;
b) die aus dem Gen *tat* der Viren HIV-2 stammenden homologen Sequenzen und die diesen Sequenzen komplementären Sequenzen, die den Positionen 8311-8338, 8363-8390, 8426-8453, 8480-8507 des Genoms des Virus HIV-2 ROD entsprechen;
c) die aus dem Gen *pol* der Viren HIV-1 und insbesondere der Viren HIV-1 BRU, HIV-1 ARV, HIV-1 HXB2, HIV-1 BH5, HIV-1 ELI, HIV-1 NDK, HIV-1 MAL, HIV-1 SF2 stammenden homologen Sequenzen und die diesen Sequenzen komplementären Sequenzen, die den Positionen 3208-3235, 3436-3463, 3647-3675, 3739-3766 des Genoms des Virus HIV-1 BRU entsprechen;
d) die aus dem Gen *pol* der Viren HIV-2 stammenden homologen Sequenzen und die diesen Sequenzen komplementären Sequenzen, die den Positionen 3141-3168, 3241-3268, 3651-3678, 3693-3720 des Genoms des Virus HIV-2 ROD entsprechen; und
e) die aus dem Gen *gag* in HIV-1 und insbesondere der Viren HIV-1 BRU, HIV-1 ARV, HIV-1 HXB2, HIV-1 BH5, HIV-1 ELI, HIV-1 NDK, HIV-1 MAL, HIV-1 SF2 stammenden homologen Sequenzen und die diesen Sequenzen komplementären Sequenzen, die den Positionen 882-909, 949-976 und 1047-1074 des Genoms des Virus HIV-1 BRU entsprechen.

2. Verfahren zum Erhalt eines Oligonukleotids nach Anspruch 1, dadurch **gekennzeichnet,** daß man ein Oligonukleotid herstellt, das einer der folgenden Formeln entspricht:

(1) 5' CAG TAG ATC CTA GAC TAG AGC CCT GGA A (Sequenz ID Nr. 1)
(2) 5' GCA TCC AGG AAG TCA GCC TAG GAC TGC T (Sequenz ID Nr. 2)
(3) 5' TTC CTG CCA TAG GAG ATG CCT AAG GCT T (Sequenz ID Nr. 3)
(4) 5' TGA GGA GGT CTT CGT CGC TGT CTC CGC T (Sequenz ID Nr. 4)
(5) 5' TGC CCA CAC TAA TGA TGT AAA ACA ATT A (Sequenz ID Nr. 5)
(6) 5' AGG AGC AGA AAC TTT CTA TGT AGA TCG G (Sequenz ID Nr. 6)
(7) 5' ATT CAC TTT TAT CTG GTT GTG CTT CAA T (Sequenz ID Nr. 7)
(8) 5' ATT TCC TCC AAT TCC TTT GTG TCC TGG T (Sequenz ID Nr. 8)
(9) 5' ATA TCC ACA AGG ACC GGG CAC AGC CAG C (Sequenz ID Nr. 9)
(10) 5' TCC AAG CAA CGG TGG AGA CAC ATA CTG G (Sequenz ID Nr. 10)
(11) 5' TAG TCT GGT CAA GAG GCG AAT CAC CTG G (Sequenz ID Nr. 11)
(12) 5' GAT GAG TTG GAG GGT CAG GAA GCT CCT G (Sequenz ID Nr. 12)
(13) 5' TCA ATG ACA TCC AGA AGC TAG TCG CTC T (Sequenz ID Nr. 13)
(14) 5' CAC ACT CAC AGA AGA AGT ACA GTG CAC A (Sequenz ID Nr. 14)
(15) 5' ATA CGA TCC CCT ACC AGG TTA AAC GCT A (Sequenz ID Nr. 15)
(16) 5' CTA TTG CAC GAT CCA TCT CTG TAG AAG G (Sequenz ID Nr. 16)
(17) 5' GAT TTA AAC ACC ATG CTA AAC ACA GTG G (Sequenz ID Nr. 17)
(18) 5' TCA ATG AGG AAG CTG CAG AAT GGG ATA G (Sequenz ID Nr. 18)
(19) 5' TTT GTT CCT GAA GGG TAC TAG TAG TTC C (Sequenz ID Nr. 19)

3. Verfahren zum Erhalt eines Diagnosereagens für die Detektion mindestens eines Retrovirus vom Typ HIV (HIV-1 und/oder HIV-2), dadurch **gekennzeichnet,** daß man das Reagens, ausgehend von mindestens einem Oligonukleotid nach Anspruch 1 oder Anspruch 2, herstellt und das Oligonukleotid gegebenenfalls in geeigneter Weise markiert.

4. Verfahren zur Herstellung eines Reagens nach Anspruch 3, dadurch **gekennzeichnet,** daß man das Reagens, ausgehend von Starterpaaren, für die Synthese eines Nukleinsäurefragments von HIV herstellt.

5. Verfahren zum Erhalt eines Reagens nach Anspruch 4, dadurch **gekennzeichnet,** daß die Starterpaare unter den folgenden Starterpaaren selektioniert werden:
. Paar A : Oligonukleotid (1) und Oligonukleotid (4),
. Paar B : Oligonukleotid (5) und Oligonukleotid (8),
. Paar C : Oligonukleotid (9) und Oligonukleotid (12),
. Paar D : Oligonukleotid (13) und Oligonukleotid (16),
. Paar E : Oligonukleotid (2) und Oligonukleotid (3),
. Paar F : Oligonukleotid (6) und Oligonukleotid (7),
. Paar G : Oligonukleotid (10) und Oligonukleotid (11),
. Paar H : Oligonukleotid (14) und Oligonukleotid (15),
. Paar I : Oligonukleotid (17) und Oligonukleotid (20), der Formel 5' TTT GGT CCC TGT CTT ATG TCC AAA ATG C (Sequenz ID Nr. 20)
. Paar J : Oligonukleotid (18) und Oligonukleotid (19).

6. Verfahren zum Erhalt eines Reagens nach Anspruch 3, dadurch **gekennzeichnet,** daß man das Reagens, ausgehend von einer Sonde für die Detektion oder den Nachweis eines Nukleisäurefragments von HIV herstellt und die Sonde in geeigneter Weise markiert.

7. Verfahren zur Detektion eines HIV-Virus durch Detektion seiner Nukleinsäuresequenz in einer biologischen Probe, dadurch **gekennzeichnet,** daß es nach geeignetem Inlösungbringen der biologischen Probe, um die Nukleinsäure zu extrahieren, umfaßt:
A. eine Stufe, in der man die biologische Probe mit mindestens einem Diagnosereagens in Berührung bringt, das aus der Gruppe ausgewählt ist, die die Reagentien gemäß einem der Ansprüche 3 bis 6 und jedes andere Reagens, das mit der zu detektierenden Sequenz hybridisieren kann, umfaßt, und
B. eine Stufe, in der man mit jedem geeigneten Mittel das/die Produkt/Produkte detektiert, die aus der Wechselwirkung zwischen gegebenenfalls vorhandener Nukleotidsequenz des HIV und dem Diagnosereagens resultieren.

8. Verfahren nach Anspruch 7, dadurch **gekennzeichnet,** daß das Diagnosereagens der Stufe A aus der Gruppe ausgewählt wird, die die Starterpaare, von denen mindestens eines ein oligonukleotid nach Anspruch 1 oder Anspruch 2 ist und die für die Amplifikation der Nukleotidsequenz des HIV geeigneten Starterpaare umfaßt und daß das Verfahren ermöglicht, das Amplifikationsprodukt der zu detektierenden Nukleotidsequenz zu erhalten.

9. Verfahren nach Anspruch 8, dadurch **gekennzeichnet,** daß die in A erhaltenen Produkte der Amplifikation durch Hybridisierung dieser Produkte der Amplifikation mit einem Diagnosereagens nach einem der Ansprüche 3 bis 6, in geeigneter Weise markiert, detektiert werden.

10. Verfahren nach Anspruch 7, dadurch **gekennzeichnet,** daß das Diagnosereagens der Stufe A eine Sonde für die Detektion oder den Nachweis nach Anspruch 3 oder Anspruch 6 ist und das Verfahren den Erhalt von Hybriden zwischen der nachzuweisenden Nukleotidsequenz und der genannten Sonde ermöglicht.

11. Verfahren nach Anspruch 7, bei dem eine Technik der genomischen Amplifikation der genannten Viren im Verlauf der vorgenannten Stufe A angewandt wird, wobei das Verfahren nach geeignetem Inlösungbringen der biologischen Probe, um die Nukleinsäure zu extrahieren, die Detektion der Nu-kleinsäuresequenz des gesuchten HIV mit Hilfe der folgenden stufen umfaßt:
(1) als Stufe A, eine Stufe der Anreicherung an Zielsequenz(en) durch:
(a) Inkontaktbringen der in Lösung gebrachten biologischen Probe mit mindestens einem Paar geeigneter Starter, um mindestens ein Fragment der genannten Nukleinsäure-Zielsequenz zu amplifizieren, wobei die Starter mit der Zielsequenz hybridisieren und erlauben, eine Anreicherungslösung zu erhalten; und
(b) mindestens eine geeignete Verdünnung der in (a) erhaltenen Amplifikations-Anreicherungslösung;
(2) eine Stufe B der Detektion der erhaltenen amplifizierten Zielsequenzen durch:
(c) Inkontaktbringen einer Fraktion der in (b) erhaltenen Anreicherungslosung mit mindestens einem Starterpaar, von denen mindestens eine der Sequenzen in die in (a) amplifizierte zielsequenz eingeschlossen ist; und
(d) Detektion der in (c) erhaltenen Kopien doppelsträngiger Zielnukleinsäure mit jedem geeigneten Mittel, dadurch **gekennzeichnet,** daß man für die Stufe (a) mindestens ein Oligonukleotid nach Anspruch 1 oder Anspruch 2 einsetzt, das mit einem Oligonukleotid gepaart ist, ausgewählt aus der Gruppe, umfassend die Oligonukleotide nach Anspruch 1 und Anspruch 2 und jedes andere Oligonukleotid, das mit den genannten Zielsequenzen hybridisieren kann, und daß man für die Stufe (c) ein Paar von Oligonukleotiden (Startern) einsetzt, die aus der Gruppe ausgewählt sind, welche die Reagentien nach einem der Ansprüche 3 bis 5 umfaßt, deren Sequenz der in (a) amplifizierten Zielsequenz homolog oder komplementär ist, sowie jedes andere Oligonukleotid, das mit den in (a) erhaltenen Zielsequenzen hybridisieren kann.

12. Verfahren nach Anspruch 11, dadurch **gekennzeichnet,** daß die Starterpaare unter folgenden Paaren ausgewählt werden:
. Paar A : Oligonukleotid (1) und Oligonukleotid (4),
. Paar B : Oligonukleotid (5) und Oligonukleotid (8),
. Paar C : Oligonukleotid (9) und Oligonukleotid (12),
. Paar D : Oligonukleotid (13) und oligonukleotid (16),
. Paar E : Oligonukleotid (2) und Oligonukleotid (3),
. Paar F : Oligonukleotid (6) und Oligonukleotid (7),
. Paar G : Oligonukleotid (10) und Oligonukleotid (11),
. Paar H : Oligonukleotid (14) und Oligonukleotid (15),
. Paar I : Oligonukleotid (17) und Oligonukleotid (20), (Sequenz ID Nr. 20) der Formel 5' TTT GGT CCC TGT CTT ATG TCC AAA ATG C
. Paar J : Oligonukleotid (18) und Oligonukleotid (19).

13. Verfahren nach Anspruch 11 oder Anspruch 12, dadurch **gekennzeichnet,** daß die Starterpaare der Stufe (a) und die Starterpaare der Stufe (c) vorzugsweise entsprechend den folgenden Gruppen assoziiert sind:
Gruppe 1 : Paar A - Paar E
Gruppe 2 : Paar B - Paar F
Gruppe 3 : Paar C - Paar G
Gruppe 4 : Paar D - Paar H
Gruppe 5 : Paar I - Paar J

14. Verfahren nach einem der Ansprüche 11 bis 13, dadurch **gekennzeichnet,** daß die Starterpaare der Stufe (c) Paare sind aus Starter, modifiziert mit einem Einfangsystem, und Starter, modifiziert mit einem De-tektionssystem.

15. Verfahren zur Herstellung eines Kit für die Detektion eines HIV-Virus, dadurch **gekennzeichnet,** daß man die für die Hybridisierung, die Detektion und gegebenenfalls die Amplifikation geeigneten Puffer und Reagentien mit mindestens einem Reagens, erhalten nach einem der Ansprüche 3 bis 6, assoziiert.

## Claims (Claims for the following Contracting State(s): BE, CH, LI, DE, FR, GB, IT)

1. Oligonucleotides comprising approx. 25 to 35 nucleotides for the differential diagnosis of HIV-1 and HIV-2 viruses, characterised in that they are selected from at least one of the following sequences:
a) the homologous sequences issuing from the tat gene of the HIV-1 and more especially the HIV-1 BRU, HIV-1 ARV, HIV-1 HXB2, HIV-1 BH5, HIV-1 ELI, HIV-1 NDK, HIV-1 MAL, HIV-1 SF2 viruses and complementary sequences, which correspond to the positions 5419-5446, 5447-5474, 5533-5560, 5563-5590 of the genome of the virus HIV-1 BRU;
b) the homologous sequences issuing from the tat gene of the HIV-2 viruses and complementary sequences, which correspond to the positions 8311-8338, 8363-8390, 8426-8453, 8480-8507 of the genome of the virus HIV-2 ROD;
c) the homologous sequences issuing from the pol gene of the HIV-1 and more especially the HIV-1 BRU, HIV-1 ARV, HIV-1 HXB2, HIV-1 BH5, HIV-1 ELI, HIV-1 NDK, HIV-1 MAL, HIV-1 SF2 viruses and complementary sequences, which correspond to the positions 3208-3235, 3436-3463, 3647-3675, 3739-3766 of the genome of the virus HIV-1 BRU;
d) the homologous sequences issuing from the pol gene of the HIV-2 viruses and complementary sequences, which correspond to the positions 3141-3168, 3241-3268, 3651-3678, 3693-3720 of the genome of the virus HIV-2 ROD, and
e) the homologous sequences issuing from the gag gene of the HIV-1 and more especially the HIV-1 BRU, HIV-1 ARV, HIV-1 HXB2, HIV-1 BH5, HIV-1 ELI, HIV-1 NDK, HIV-1 MAL, HIV-1 SF2 viruses and complementary sequences and which correspond to the positions 882-909, 949-976 and 1047-1074 of the genome of the virus HIV-1 BRU.

2. Oligonucleotides according to Claim 1, characterised in that they comply with any one of the following formulae:

(1) 5' CAG TAG ATC CTA GAC TAG AGC CCT GGA A (SEQUENCE ID N° 1)

(2) 5' GCA TCC AGG AAG TCA GCC TAG GAC TGC T (SEQUENCE ID N° 2)

(3) 5' TTC CTG CCA TAG GAG ATG CCT AAG GCT T (SEQUENCE ID N° 3)

(4) 5' TGA GGA GGT CTT CGT CGC TGT CTC CGC T (SEQUENCE ID N° 4)

(5) 5' TGC CCA CAC TAA TGA TGT AAA ACA ATT A (SEQUENCE ID Nº 5)

(6) 5' AGG· AGC AGA AAC TTT CTA TGT AGA TGG G (SEQUENCE ID N° 6)

(7) 5' ATT CAC TTT TAT CTG GTT GTG CTT GAA T (SEQUENCE ID N° 7)

(8) 5' ATT TCC TCC AAT TCC TTT GTG TGC TGG T (SEQUENCE ID N° 8)

(9) 5' ATA TCC ACA AGG ACC GGG GAC AGC CAG C (SEQUENCE ID N° 9)

(10) 5' TGG AAG CAA CGG TGG AGA CAG ATA CTG G (SEQUENCE ID N° 10)

(11) 5' TAG TCT GGT CAA GAG GCG AAT CAG CTG G (SEQUENCE ID N° 11)

(12) 5' GAT GAG TTG GAG GGT CAG GAA GCT CCT G (SEQUENCE ID N° 12)

(13) 5' TCA ATG ACA TCC AGA AGC TAG TGG GTG T (SEQUENCE ID N° 13)

(14) 5' GAC ACT CAC AGA AGA AGT ACA GTG GAC A (SEQUENCE ID N° 14)

(15) 5' ATA GGA TCC CCT ACC AGG TTA AAC GCT A (SEQUENCE ID N° 15)

(16) 5' CTA TTG CAG GAT CCA TCT GTG TAG AAG G (SEQUENCE ID N° 16)

(17) 5' GAT TTA AAC ACC ATG CTA AAC ACA GTG G (SEQUENCE ID N° 17)

(18) 5' TCA ATG AGG AAG CTG CAG AAT GGG ATA G (SEQUENCE ID N° 18)

(19) 5' TTT GTT CCT GAA GGG TAC TAG TAG TTC C (SEQUENCE ID N° 19)

3. Diagnostic reagent for the detection of at least one Type HIV (HIV-1 and/or HIV-2) retrovirus, characterised in that it comprises at least one oligonucleotide according to Claim 1 or Claim 2, possibly marked in a suitable manner.

4. A reagent according to Claim 3, characterised in that it corresponds to pairs of primers for synthesising a nucleic fragment of HIV.

5. A reagent according to Claim 4, characterised in that it is in particular constituted by one of the following pairs of primers:
- pair A: oligonucleotide (1) and oligonucleotide (4),
- pair B: oligonucleotide (5) and oligonucleotide (8),
- pair C: oligonucleotide (9) and oligonucleotide (12),
- pair D: oligonucleotide (13) and oligonucleotide (16),
- pair E: oligonucleotide (2) and oligonucleotide (3),
- pair F: oligonucleotide (6) and oligonucleotide (7),
- pair G: oligonucleotide (10) and oligonucleotide (11),
- pair H: oligonucleotide (14) and oligonucleotide (15),
- pair I: oligonucleotide (17) and oligonucleotide (20), of formula 5' TTT GGT CCC TGT CTT ATG TCC AAA ATG C (SEQUENCE ID No. 20)
- pair J: oligonucleotide (18) and oligonucleotide (19).

6. A reagent according to Claim 3, characterised in that it corresponds to a probe for detecting or revealing a nucleic fragment of HIV and is appropriately marked.

7. A method of detecting an HIV virus by detecting its nucleic acid sequence in a biological sample, characterised in that, once the biological sample has been brought to a solution for extraction of the nucleic acid, it comprises:
A. a stage in which the biological sample is brought into contact with at least one diagnostic reagent chosen from the group which comprises the reagents according to any one of Claims 3 to 6 and any other reagent adapted to hybridise with the sequence to be detected, and
B. a stage in which by any suitable means the product(s) resulting from the interaction of the diagnostic reagent and the possibly present nucleotidic HIV sequence.

8. A method according to Claim 7, characterised in that the diagnostic reagent of stage A is chosen from the group which comprises the pairs of primers in which at least one of them is an oligonucleotide according to Claim 1 or Claim 2 and the pairs of primers suitable for amplification of the nucleotidic HIV sequence, the said method making it possible to obtain the product of amplification of the nucleotidic sequence to be detected.

9. A method according to Claim 8, characterised in that the products of amplification obtained in A are detected by hybridisation among the said products of amplification and a diagnostic reagent according to any one of Claims 3 to 6, marked in a suitable manner.

10. A method according to Claim 7, characterised in that the diagnostic reagent of stage A is a detection or revelation probe according to Claim 3 or Claim 6, the said method making it possible to obtain hybrids between the said probe and the nucleotidic sequence to be detected.

11. A method according to Claim 7, employing a technique for the genomic amplification of the said viruses during the course of the aforesaid stage A, whereby, after the biological sample has been suitably brought into solution in order to extract the nucleic acid, the said method includes detection of the nucleic acid sequence of the HIV being sought, by means of the following stages:
(1) like stage A, a stage of enrichment with target sequence(s) by:
(a) bringing the biological sample, in solution, into contact with at least one pair of suitable primers in order to amplify at least one fragment of the said target nucleic acid sequence, the said primers hybridising with the said target sequence and making it possible to obtain an enrichment solution, and
(b) at least one appropriate dilution of the enrichment amplification solution obtained in (a);
(2) and a stage B for detecting the amplified target sequences by:
(c) bringing a fraction of the enrichment solution obtained in (b) in contact with at least one pair of primers of which at least one of the sequences is included in the target sequence amplified in (a); and
(d) detecting copies of double strand target nucleic acid obtained in (c) by any appropriate means, the said method being characterised in that for stage (a), at least one oligonucleotide according to Claim 1 or Claim 2 is used, paired with an oligonucleotide chosen from the group which comprises the oligonucleotides according to Claim 1 and Claim 2 and any other nucleotide capable of hybridising with the said target sequences and for stage (c), a pair of nucleotides (primers) chosen from the group which comprises the reagents according to any one of Claims 3 to 5, the sequence of which is homologous with or complementary to the target sequence amplified in (a) and any other oligonucleotide capable of hybridising with the target sequences obtained in (a).

12. A method according to Claim 11, characterised in that the pairs of primers are preferably selected from the following pairs:
- pair A: oligonucleotide (1) and oligonucleotide (4),
- pair B: oligonucleotide (5) and oligonucleotide (8),
- pair C: oligonucleotide (9) and oligonucleotide (12),
- pair D: oligonucleotide (13) and oligonucleotide (16),
- pair E: oligonucleotide (2) and oligonucleotide (3),
- pair F: oligonucleotide (6) and oligonucleotide (7),
- pair G: oligonucleotide (10) and oligonucleotide (11),
- pair H: oligonucleotide (14) and oligonucleotide (15),
- pair I: oligonucleotide (17) and oligonucleotide (20), of formula 5' TTT GGT CCC TGT CTT ATG TCC AAA ATG C (sequence ID No. 20),
- pair J: oligonucleotide (18) and oligonucleotide (19).

13. A method according to Claim 11 or Claim 12, characterised in that the pairs of primers from stage (a) and the pairs of primers from stage (c) are preferably associated according to the following groups:
group 1 : pair A - pair E
group 2 : pair B - pair F
group 3 : pair C - pair G
group 4 : pair D - pair H
group 5 : pair I - pair J

14. A method according to any one of Claims 11 to 13, characterised in that the pairs of primers from stage (c) are pairs : primer modified by a capture system - primer modified by a detection system.

15. A kit for the detection of an HIV virus, characterised in that, in addition to the buffers and reagents appropriate to hybridisation, detection and possibly amplification, it comprises a reagent according to any one of Claims 3 to 6.

## Claims (Claims for the following Contracting State(s): ES)

1. A method of obtaining an oligonucleotide comprising approx. 25 to 35 nucleotides for the differential diagnosis of HIV-1 and HIV-2 viruses, characterised in that at least one of the following sequences is prepared and selected:
a) the homologous sequences issuing from the *tat* gene of the HIV-1 and more especially the HIV-1 BRU, HIV-I ARV, HIV-1 HXB2, HIV-1 BH5, HIV-1 ELI, HIV-1 NDK, HIV-1 MAL, HIV-1 SF2 viruses and complementary sequences, which correspond to the positions 5419-5446, 5447-5474, 5533-5560, 5563-5590 of the genome of the virus HIV-1 BRU;
b) the homologous sequences issuing from the *tat* gene of the HIV-2 viruses and complementary sequences, which correspond to the positions 8311-8338, 8363-8390, 8426-8453, 8480-8507 of the genome of the virus HIV-2 ROD;
c) the homologous sequences issuing from the *pol* gene of the HIV-1 and more especially the HIV-1 BRU, HIV-1 ARV, HIV-1 HXB2, HIV-1 BH5, HIV-I ELI, HIV-1 NDK, HIV-1 MAL, HIV-1 SF2 viruses and complementary sequences, which correspond to the positions 3208-3235, 3436-3463, 3647-3675, 3739-3766 of the genome of the virus HIV-1 BRU;
d) the homologous sequences issuing from the *pol* gene of the HIV-2 viruses and complementary sequences, which correspond to the positions 3141-3168, 3241-3268, 3651-3678, 3693-3720 of the genome of the virus HIV-2 ROD, and
e) the homologous sequences issuing from the *gag* gene of the HIV-1 and more especially the HIV-1 BRU, HIV-1 ARV, HIV-1 HXB2, HIV-1 BH5, HIV-1 ELI, HIV-1 NDK, HIV-1 MAL, HIV-1 SF2 viruses and the sequences which are complementary to these sequences and which correspond to the positions 882-909, 949-976 and 1047-1074 of the genome of the virus HIV-1 BRU.

2. A method of obtaining an oligonucleotide according to Claim 1, characterised in that an oligonucleotide is prepared which complies with any one of the following formulae:

(1) 5' CAG TAG ATC CTA GAC TAG AGC CCT GGA A (SEQUENCE ID N° 1)

(2) 5' GCA TCC AGG AAG TCA GCC TAG GAC TGC T (SEQUENCE ID N° 2)

(3) 5' TTC CTG CCA TAG GAG ATG CCT AAG GCT T (SEQUENCE ID N° 3)

(4) 5' TGA GGA GGT CTT CGT CGC TGT CTC CGC T (SEQUENCE ID N° 4)

(5) 5' TGC CCA CAC TAA TGA TGT AAA ACA ATT A (SEQUENCE ID N° 5)

(6) 5' AGG· AGC AGA AAC TTT CTA TGT AGA TGG G (SEQUENCE ID N° 6)

(7) 5' ATT CAC TTT TAT CTG GTT GTG CTT GAA T (SEQUENCE ID N° 7)

(8) 5' ATT TCC TCC AAT TCC TTT GTG TGC TGG T (SEQUENCE ID N° 8)

(9) 5' ATA TCC ACA AGG ACC GGG GAC AGC CAG C (SEQUENCE ID N° 9)

(10) 5' TGG AAG CAA CGG TGG AGA CAG ATA CTG G (SEQUENCE ID N° 10)

(11) 5' TAG TCT GGT CAA GAG GCG AAT CAG CTG G (SEQUENCE ID N° 11)

(12) 5' GAT GAG TTG GAG GGT CAG GAA GCT CCT G (SEQUENCE ID N° 12)

(13) 5' TCA ATG ACA TCC AGA AGC TAG TGG GTG T (SEQUENCE ID N° 13)

(14) 5' GAC ACT CAC AGA AGA AGT ACA GTG GAC A (SEQUENCE ID N° 14)

(15) 5' ATA GGA TCC CCT ACC AGG TTA AAC GCT A (SEQUENCE ID N° 15)

(16) 5' CTA TTG CAG GAT CCA TCT GTG TAG AAG G (SEQUENCE ID N° 16)

(17) 5' GAT TTA AAC ACC ATG CTA AAC ACA GTG G (SEQUENCE ID N° 17)

(18) 5' TCA ATG AGG AAG CTG CAG AAT GGG ATA G (SEQUENCE ID N° 18)

(19) 5' TTT GTT CCT GAA GGG TAC TAG TAG TTC C (SEQUENCE ID N° 19)

3. A method of obtaining a diagnostic reagent for the detection of at least one Type HIV (HIV-1 and/or HIV-2) retrovirus, characterised in that the said reagent is prepared from at least one oligonucleotide according to Claim 1 or Claim 2, and in that the said nucleotide is possibly marked in a suitable manner.

4. A method of obtaining a reagent according to Claim 3, characterised in that the said reagent is prepared from pairs of primers for synthesising a nucleic fragment of HIV.

5. A method of obtaining a reagent according to Claim 4, characterised in that the pairs of primers are selected from the following pairs of primers:
- pair A: oligonucleotide (1) and oligonucleotide (4),
- pair B: oligonucleotide (5) and oligonucleotide (8),
- pair C: oligonucleotide (9) and oligonucleotide (12),
- pair D: oligonucleotide (13) and oligonucleotide (16),
- pair E: oligonucleotide (2) and oligonucleotide (3),
- pair F: oligonucleotide (6) and oligonucleotide (7),
- pair G: oligonucleotide (10) and oligonucleotide (11),
- pair H: oligonucleotide (14) and oligonucleotide (15),
- pair I: oligonucleotide (17) and oligonucleotide (20), of formula 5' TTT GGT CCC TGT CTT ATG TCC AAA ATG C (SEQUENCE ID No. 20)
- pair J: oligonucleotide (18) and oligonucleotide (19).

6. A method of obtaining a reagent according to Claim 3, characterised in that the said reagent is prepared from a probe for detecting or revealing a nucleic fragment of HIV and in that the said probe is appropriately marked.

7. A method of detecting an HIV virus by detecting its nucleic acid sequence in a biological sample, characterised in that, once the biological sample has been brought to a solution for extraction of the nucleic acid, it comprises:
A. a stage in which the biological sample is brought into contact with at least one diagnostic reagent chosen from the group which comprises the reagents according to any one of Claims 3 to 6 and any other reagent adapted to hybridise with the sequence to be detected, and
B. a stage in which by any suitable means the product(s) resulting from the interaction of the diagnostic reagent and the possibly present nucleotidic HIV sequence.

8. A method according to Claim 7, characterised in that the diagnostic reagent of stage A is chosen from the group which comprises the pairs of primers in which at least one of them is an oligonucleotide according to Claim 1 or Claim 2 and the pairs of primers suitable for amplification of the nucleotidic HIV sequence, the said method making it possible to obtain the product of amplification of the nucleotidic sequence to be detected.

9. A method according to Claim 8, characterised in that the products of amplification obtained in A are detected by hybridisation among the said products of amplification and a diagnostic reagent according to any one of Claims 3 to 6, marked in a suitable manner.

10. A method according to Claim 7, characterised in that the diagnostic reagent of stage A is a detection or revelation probe according to Claim 3 or Claim 6, the said method making it possible to obtain hybrids between the said probe and the nucleotidic sequence to be detected.

11. A method according to Claim 7, employing a technique for the genomic amplification of the said viruses during the course of the aforesaid stage A, whereby, after the biological sample has been suitably brought into solution in order to extract the nucleic acid, the said method includes detection of the nucleic acid sequence of the HIV being sought, by means of the following stages:
(1) like stage A, a stage of enrichment with target sequence(s) by:
(a) bringing the biological sample, in solution, into contact with at least one pair of suitable primers in order to amplify at least one fragment of the said target nucleic acid sequence, the said primers hybridising with the said target sequence and making it possible to obtain an enrichment solution, and
(b) at least one appropriate dilution of the enrichment amplification solution obtained in (a);
(2) and a stage B for detecting the amplified target sequences by:
(c) bringing a fraction of the enrichment solution obtained in (b) in contact with at least one pair of primers of which at least one of the sequences is included in the target sequence amplified in (a); and
(d) detecting copies of double strand target nucleic acid obtained in (c) by any appropriate means, the said method being characterised in that for stage (a), at least one oligonucleotide according to Claim I or Claim 2 is used, paired with an oligonucleotide chosen from the group which comprises the oligonucleotides according to Claim 1 and Claim 2 and any other nucleotide capable of hybridising with the said target sequences and for stage (c), a pair of nucleotides (primers) chosen from the group which comprises the reagents according to any one of Claims 3 to 5, the sequence of which is homologous with or complementary to the target sequence amplified in (a) and any other oligonucleotide capable of hybridising with the target sequences obtained in (a).

12. A method according to Claim 11, characterised in that the pairs of primers are preferably selected from the following pairs:
- pair A: oligonucleotide (1) and oligonucleotide (4),
- pair B: oligonucleotide (5) and oligonucleotide (8),
- pair C: oligonucleotide (9) and oligonucleotide (12),
- pair D: oligonucleotide (13) and oligonucleotide (16),
- pair E: oligonucleotide (2) and oligonucleotide (3),
- pair F: oligonucleotide (6) and oligonucleotide (7),
- pair G: oligonucleotide (10) and oligonucleotide (11),
- pair H: oligonucleotide (14) and oligonucleotide (15),
- pair I: oligonucleotide (17) and oligonucleotide (20), of formula 5' TTT GGT CCC TGT CTT ATG TCC AAA ATG C (sequence ID No. 20),
- pair J: oligonucleotide (18) and oligonucleotide (19).

13. A method according to Claim 11 or Claim 12, characterised in that the pairs of primers from stage (a) and the pairs of primers from stage (c) are preferably associated according to the following groups:
group 1 : pair A - pair E
group 2 : pair B - pair F
group 3 : pair C - pair G
group 4 : pair D - pair H
group 5 : pair I - pair J

14. A method according to any one of Claims 11 to 13, characterised in that the pairs of primers from stage (c) are pairs : primer modified by a capture system - primer modified by a detection system.

15. A method of preparing a kit for the detection of an HIV virus, characterised in that the buffers and reagents appropriate to hybridisation, detection and possibly amplification are associated with a reagent according to any one of Claims 3 to 6.
